# EUROPEAN PATENT APPLICATION

(11) **EP 4 385 435 A1**
(43) Date of publication of application: **19.06.2024**
(21) Application number: 22855462.2
(22) Date of filing: 10.08.2022
(51) Int. Cl.: A61B 18/00, H02M 3/156, H02M 3/335

(54) **HIGH-VOLTAGE GENERATING CIRCUIT FOR CATHETER AND ABLATION TOOL**

(30) Priority: 10.08.2021 CN 202110912278; 10.08.2021 CN 202110912538
(71) Applicant: Accupulse Medical Technology (Suzhou) Co., Ltd., Jiangsu 215127 (CN)
(72) Inventor: ZHAO, Chenggang, Suzhou, Jiangsu 215127 (CN)
(74) Representative: Lavoix
(86) International application number: PCT/CN2022/111390
(87) International publication number: WO 2023/016482

(57) **Abstract**

A high-voltage generating circuit for a catheter and an ablation tool. The high-voltage generating circuit comprises: N voltage conversion units (111a, 121a, 131a, 141a), N energy storage units (112a, 122a, 132a, 142a), a voltage adjustment unit (200a), and a control unit (300a). Input ends of the N voltage conversion units (111a, 121a, 131a, 141a) are connected in parallel and then connected to a total input end, and output ends of the N voltage conversion units (111a, 121a, 131a, 141a) are connected in series and then connected to a total output end; the N energy storage units (112a, 122a, 132a, 142a) have one-to-one correspondence to the N voltage conversion units (111a, 121a, 131a, 141a); an input end of the voltage adjustment unit (200a) is connected to the output ends of the voltage conversion units (111a 121a 131a 141a) and an output end of the voltage adjustment unit (200a) is connected to the total output end: and the control unit (300a) is connected to the N voltage conversion units (111a, 121a, 131a, 141a) and the voltage adjustment unit (200a) so as to adjust the output voltage of the total output end to be a target voltage. In this way, the output voltage can be quickly switched to the target voltage by means of the multiple input-parallel output-series voltage conversion units (111a, 121a 131a, 141a), and meanwhile, the output range of the voltage is widened, such that the application prospect of a pulsed electric field ablation technology in treatment of arrhythmia is improved.

## Description

### TECHNICAL FIELD

The present application relates to the technical field of pulsed electric field ablation, and in particular to a high-voltage generating circuit for a catheter and an ablation tool.

### BACKGROUND ART

Generally, the ablation energy in the catheter ablation technology currently used to treat arrhythmias is mainly based on radiofrequency energy, supplemented by cryoenergy. These two ablation methods have shown certain advantages in the treatment of arrhythmias, but they also have corresponding limitations. For example, ablation energy lacks selectivity in destroying tissues in the ablation area, and relies on the adhesion force of the catheter, which may cause certain damage to the adjacent esophagus, coronary arteries, and phrenic nerves. Therefore, relevant technologies to find a fast, safe, and efficient ablation energy to complete and achieve durable pulmonary vein isolation without damaging adjacent tissues have become a research focus.

Pulsed electric field ablation technology is a new ablation method that uses pulsed electric field as energy. As a non-thermal ablation technology, it has increasingly attracted attention in clinical applications. Pulsed electric field ablation technology mainly generates a high-voltage pulsed electric field with a pulse width of milliseconds, microseconds or even nanoseconds to release extremely high energy in a short period of time, so that a large number of irreversible micropores will be generated on cell membranes and even intracellular organelles such as the endoplasmic reticulum, mitochondria, cell nuclei, etc., which will cause the apoptosis of diseased cells, thereby achieving the expected therapeutic purpose. However, as a new energy ablation technology, pulsed electric field ablation technology has the defect that the output voltage of the high-voltage generating circuit cannot be quickly switched to the target voltage, thereby limiting the clinical application of pulsed electric field ablation technology.

### CONTENTS OF THE APPLICATION

The present application aims to solve one of the technical problems in the related technology, at least to a certain extent.

To this end, the first object of the present application is to provide a high-voltage generating circuit for a catheter and a corresponding ablation tool. The circuit can quickly switch the output voltage to the target voltage by using a plurality of input-parallel and output-series voltage conversion units, and meanwhile, the output range of the voltage is widened, thereby improving the application prospects of pulsed electric field ablation technology in arrhythmia treatment.

In order to achieve the above first object, a first embodiment of the present application provides a high-voltage generating circuit for a catheter, comprising: N voltage conversion units and N energy storage units, wherein the input terminals of the N voltage conversion units are connected in parallel and then connected to a total input terminal of the high-voltage generating circuit, the output terminals of the N voltage conversion units are connected in series and then connected to a total output terminal of the high-voltage generating circuit; the N energy storage units correspond one-to-one to the N voltage conversion units, and each energy storage unit is connected between the output terminals of the corresponding voltage conversion units, and each of the N voltage conversion units is used to perform voltage conversion on the input voltage of the total input terminal and charge the corresponding energy storage unit, wherein N is an integer greater than 1; a voltage adjustment unit, whose input terminal is connected to an output terminal of each voltage conversion unit, wherein an output terminal of the voltage adjustment unit is connected to the total output terminal so as to control the on-off switching between the output terminal of each voltage conversion unit and the total output terminal; and a control unit, which is connected to the N voltage conversion units and the voltage adjustment unit so as to obtain a target voltage of the high-voltage generating circuit, wherein the N voltage conversion units and the voltage adjustment unit are controlled according to the target voltage so as to adjust the output voltage of the total output terminal to be a target voltage.

According to the high-voltage generating circuit for a catheter according to an embodiment of the present application, the input terminals of the voltage conversion units are connected in parallel and then connected to the total input terminal of the high-voltage generating circuit, and the output terminals of the voltage conversion units are connected in series and then connected to the total output terminal of the high-voltage generating circuit. The voltage adjustment unit is connected between the output terminal of each voltage conversion unit and the total output terminal so as to control the on-off switching between the output terminal of each voltage conversion unit and the total output terminal. The control unit is connected to each voltage conversion unit and the voltage adjustment unit so as to control the voltage conversion unit and the voltage adjustment unit to adjust the output voltage of the total output terminal to be a target voltage. As a result, the output voltage can be rapidly switched to the target voltage by using a plurality of input-parallel and output-series voltage conversion units, and meanwhile, the output range of the voltage is widened, thereby improving the application prospects of pulsed electric field ablation technology in arrhythmia treatment.

According to an implementation of the first embodiment of the present application, the N voltage conversion units cooperate with the N energy storage units to form N voltage intervals, and the control unit is specifically used for: obtaining a voltage interval corresponding to the target voltage, and wherein the N voltage conversion units and the voltage adjustment unit are controlled based on the voltage interval corresponding to the target voltage.

According to an implementation of the first embodiment of the present application, the high-voltage generating circuit for a catheter further comprises: a current adjustment unit, whose input terminal is connected to an output terminal of the voltage adjustment unit, wherein an output terminal of the current adjustment unit is connected to the total output terminal so as to adjust the output current of the total output terminal; and wherein a control unit is also connected to the current adjustment unit so as to obtain a target power of the high-voltage generating circuit; the N voltage conversion units, the voltage adjustment unit and the current adjustment unit are controlled based on the target power of the high-voltage generating circuit, so that the output voltage and output current of the total output terminal are adjusted to obtain the target power.

According to an implementation of the first embodiment of the present application, the N energy storage units have the same structure, and each energy storage unit comprises an energy storage capacitor which is connected in parallel between the output terminals of the corresponding voltage conversion units.

According to an implementation of the first embodiment of the present application, the voltage adjustment unit comprises: N first switches which correspond one-to-one to the N voltage conversion units, and each first switch is connected in series between an output terminal of the corresponding voltage conversion unit and the total output terminal.

According to an implementation of the first embodiment of the present application, the N first switches have the same structure, and each first switch comprises: a relay, wherein one end of the switch of the relay is connected to an output terminal of the corresponding voltage conversion unit, and one end of the coil of the relay is connected to the preset power supply; a voltage stabilizing tube, wherein its anode is connected to the other end of the coil of the relay and its cathode is connected to said one end of the coil of the relay; a second diode, wherein its anode is connected to the other end of the switch of the relay and its cathode is connected to the total output terminal; a second switch tube, wherein its first end is connected to the other end of the coil of the relay, and its second switch tube is connected to ground, and wherein the control terminal of the second switch tube is connected to the control unit.

According to an implementation of the first embodiment of the present application, the current adjustment unit comprises M current adjustment branches connected in parallel, M is an integer greater than 1, and each current adjustment branch comprises: a current-limiting resistor, one end of which is connected to an output terminal of the voltage adjustment unit; and a current-limiting switch, one end of which is connected to the other end of the current-limiting resistor, wherein the other end of the current-limiting switch is connected to the total output terminal.

According to an implementation of the first embodiment of the present application, the N voltage conversion units have the same structure, each voltage conversion unit comprises a switch tube for voltage conversion, and some or all of the N voltage conversion units share a switch tube.

According to an implementation of the first embodiment of the present application, each of the voltage conversion units is a flyback conversion circuit, a forward conversion circuit, an LLC resonance circuit, a push-pull circuit or a bridge circuit.

According to an implementation of the first embodiment of the present application, the control unit comprises one or more control chips, when the control unit comprises one control chip, the N voltage conversion units share the control chip; or when the control unit comprises multiple control chips, some of the N voltage conversion units share one control chip, or each voltage conversion unit corresponds to one of the control chips.

According to an implementation of the first embodiment of the present application, the high-voltage generating circuit for a catheter further comprises an overvoltage protection unit, which comprises voltage stabilizing tubes connected in parallel with each of the energy storage units.

According to an implementation of the first embodiment of the present application, the overvoltage protection unit also comprises an optical coupler, and each of the optical couplers corresponds to one control chip; wherein in each voltage conversion unit controlled by the control chip and the corresponding energy storage unit, at least two or more voltage stabilizing tubes are connected in parallel to at least an energy storage unit closest to the low voltage end, a light emitter of the optical coupler is connected in parallel with part of the voltage stabilizing tubes, and an output terminal of the light receptor of the optical coupler is connected to the under-voltage protection pin of the control chip.

A second embodiment of the present application provides an ablation tool comprising the high-voltage generating circuit for a catheter according to the first embodiment.

According to the ablation tool of the second embodiment of the present application, through the above-mentioned high-voltage generating circuit for the catheter, the output voltage can be quickly switched to a target voltage by using a plurality of input-parallel and output-series voltage conversion units, and meanwhile, the output range of the voltage is widened, thereby improving the application prospects of pulsed electric field ablation technology in arrhythmia treatment.

The second object of the present application is to provide a high-voltage generating circuit for a catheter and a corresponding ablation tool. This circuit can quickly switch the output voltage to a target voltage through multiple voltage conversion units and voltage feedback units, thereby improving the application prospects of pulsed electric field ablation technology in arrhythmia treatment.

In order to achieve the above second object, the third embodiment of the present application provides a high-voltage generating circuit for a catheter, comprising: N voltage conversion units and N energy storage units, wherein the input terminals of the N voltage conversion units are connected in parallel and then connected to a total input terminal of the high-voltage generating circuit, the output terminals of the N voltage conversion units are connected in series and then connected to a total output terminal of the high-voltage generating circuit; the N energy storage units correspond one-to-one to the N voltage conversion units, and each energy storage unit is connected between the output terminals of the corresponding voltage conversion units, and each of the N voltage conversion units is used to perform voltage conversion on the input voltage of the total input terminal and charge the corresponding energy storage unit, wherein N is an integer greater than 1; a voltage feedback unit, whose detection end is connected to the total output terminal so as to detect the output voltage of the total output terminal to obtain a feedback voltage and adjust the feedback voltage; a first control unit, which is connected to the adjustment terminal of the voltage feedback unit so as to control the voltage feedback unit to adjust the feedback voltage; a second control unit, which is connected to the output terminals of the N voltage conversion units and the voltage feedback unit so as to obtain a target voltage of the high-voltage generating circuit, wherein the N voltage conversion units are controlled based on the target voltage and the adjusted feedback voltage, so as to adjust the output voltage of the total output terminal to obtain a target voltage.

According to the high-voltage generating circuit for a catheter according to an embodiment of the present application, the input terminals of the voltage conversion units are connected in parallel and then connected to the total input terminal of the high-voltage generating circuit, and the output terminals of the voltage conversion units are connected in series and then connected to the total output terminal of the high-voltage generating circuit; wherein the detection terminal of the voltage feedback unit is connected to the total output terminal so as to detect the output voltage of the total output terminal to obtain the feedback voltage and adjust the feedback voltage; a first control unit is connected to the adjustment terminal of the voltage feedback unit so as to control the voltage feedback unit to adjust the feedback voltage; a second control unit is used to obtain a target voltage of the high-voltage generating circuit, wherein the N voltage conversion units are controlled based on the target voltage and the adjusted feedback voltage, so as to adjust the output voltage of the total output terminal to obtain a target voltage. As a result, the output voltage can be quickly switched to a target voltage through using multiple voltage conversion units and voltage feedback units, thereby improving the application prospects of pulsed electric field ablation technology in arrhythmia treatment.

According to an implementation of the third embodiment of the present application, the voltage feedback unit comprises: a voltage sampling subunit, wherein its one end is connected to the total output terminal; a voltage adjustment subunit, wherein its one end is connected to the other end of the voltage sampling subunit and forms a first node, the first node is connected to a second control unit, and the other end of the voltage adjustment subunit is connected to a first control unit.

According to an implementation of the third embodiment of the present application, the voltage sampling subunit comprises: a sampling resistor, whose one end is connected to the total output terminal; the voltage adjustment subunit comprises: a resistance-adjustable chip, whose first end is connected to the other end of the sampling resistor, wherein a second end of the resistance-adjustable chip is connected to ground, and the control terminal of the resistance-adjustable chip is connected to a first control unit.

According to an implementation of the third embodiment of the present application, a first control unit is specifically used to send an adjustment instruction of feedback voltage to the resistance-adjustable chip, so that the resistance-adjustable chip can obtain an adjustment amount of resistance according to the adjustment instruction of feedback voltage, and then adjust its own resistance based on the adjustment amount of resistance.

According to an implementation of the third embodiment of the present application, the high-voltage generating circuit for the catheter further comprises: a voltage adjustment unit, whose input terminal is connected to the output terminal of each voltage conversion unit, wherein the output terminal of the voltage adjustment unit is connected to the total output terminal so as to control the on-off switching between the output terminal of each voltage conversion unit and the total output terminal; and wherein a second control unit is also used to control the N voltage conversion units and the voltage adjustment unit based on the target voltage and the adjusted feedback voltage, so as to adjust the output voltage of the total output terminal to obtain a target voltage.

According to an implementation of the third embodiment of the present application, the N voltage conversion units cooperate with the N energy storage units to form N voltage intervals, and a second control unit is specifically used for: obtaining a voltage interval corresponding to the target voltage, and wherein the N voltage conversion units and the voltage adjustment unit are controlled based on the voltage interval corresponding to the target voltage and the adjusted feedback voltage.

According to an implementation of the third embodiment of the present application, the high-voltage generating circuit for the catheter further comprises: a current adjustment unit, whose input terminal is connected to the output terminal of the voltage adjustment unit, wherein the output terminal of the current adjustment unit is connected to the total output terminal so as to adjust the output current of the total output terminal; and wherein a second control unit is also used to obtain a target power of the high-voltage generating circuit; wherein the N voltage conversion units, the voltage adjustment unit and the current adjustment unit are controlled based on the target power and the adjusted feedback voltage, so that the output voltage and output current of the total output terminal are adjusted to obtain the target power.

According to an implementation of the third embodiment of the present application, the voltage adjustment unit comprises: N first switches, the N first switches correspond one-to-one to the N voltage conversion units, and each first switch is connected in series between the output terminal of the corresponding voltage conversion unit and the total output terminal.

According to an implementation of the third embodiment of the present application, the current adjustment unit comprises M current adjustment branches connected in parallel, and each current adjustment branch comprises: a current-limiting resistor, one end of which is connected to an output terminal of the voltage adjustment unit; and a current-limiting switch, one end of the current-limiting switch is connected to the other end of the current-limiting resistor, and the other end of the current-limiting switch is connected to the total output terminal.

According to an implementation of the third embodiment of the present application, each of the voltage conversion units is a flyback conversion circuit, a forward conversion circuit, an LLC resonance circuit, a push-pull circuit or a bridge circuit.

A fourth embodiment of the present application provides an ablation tool, which comprises the high-voltage generating circuit for a catheter according to the third embodiment.

According to the ablation tool of the fourth embodiment of the present application, through the above-mentioned high-voltage generating circuit for a catheter, the output voltage can be quickly switched to a target voltage through multiple voltage conversion units and voltage feedback units, thereby improving the application prospects of pulsed electric field ablation technology in arrhythmia treatment.

Additional aspects and advantages of the application will be set forth in part in the description which follows, and some of them will be obvious from the following description, or may be learned by practice of the application.

### BRIEF DESCRIPTION OF THE DRAWINGS

Fig. 1 is a schematic structural diagram of an implementation of a high-voltage generating circuit for a catheter according to the first embodiment of the present application;
Fig. 2 is a schematic structural diagram of another implementation of a high-voltage generating circuit for a catheter according to the first embodiment of the present application;
Fig. 3 is a schematic structural diagram of still another implementation of a high-voltage generating circuit for a catheter according to the first embodiment of the present application;
Fig. 4 is a schematic structural diagram of a first switch of a high-voltage generating circuit for a catheter according to the first embodiment of the present application;
Fig. 5 is a schematic structural diagram of yet another implementation of a high-voltage generating circuit for a catheter according to the first embodiment of the present application;
Fig. 6 is a schematic diagram of an overvoltage protection unit for a high-voltage generating circuit for a catheter according to the first embodiment of the present application;
Fig. 7 is a schematic structural diagram of an ablation tool according to the second embodiment of the present application;
Fig. 8 is a schematic structural diagram of the first implementation of a high-voltage generating circuit for a catheter according to the third embodiment of the present application;
Fig. 9 is a schematic structural diagram of the second embodiment of a high-voltage generating circuit for a catheter according to the third embodiment of the present application;
Fig. 10 is a schematic structural diagram of the third embodiment of a high-voltage generating circuit for a catheter according to the third embodiment of the present application;
Fig. 11 is a schematic structural diagram of the fourth embodiment of a high-voltage generating circuit for a catheter according to the third embodiment of the present application;
Fig. 12 is a schematic structural diagram of a first switch of a high-voltage transmitting circuit for a catheter according to a third embodiment of the present application;
Fig. 13 is a schematic structural diagram of an ablation tool according to the fourth embodiment of the present application.

### DETAILED DESCRIPTION

Embodiments of the present application are described in detail below, and examples of which are illustrated in the accompanying drawings, wherein the same or similar reference numerals throughout represent the same or similar elements or elements with the same or similar functions. The embodiments described below with reference to the drawings are exemplary, and are intended to explain the present application and should not to be construed as limiting the present application.

The high-voltage generating circuit for a catheter and an ablation tool provided by embodiments of the present application will be described below with reference to the accompanying drawings.

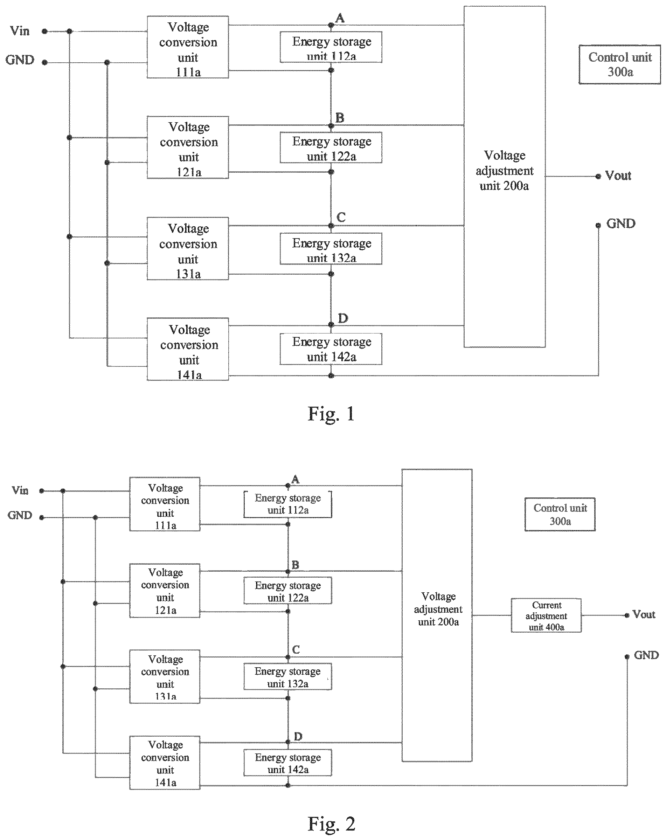

Fig. 1 is a schematic structural diagram of a high-voltage generating circuit for a catheter according to the first embodiment of the present application. Referring to Fig. 1, the high-voltage generating circuit may comprise: N voltage conversion units (N is an integer greater than 1), N energy storage units, voltage adjustment unit 200a and control unit 300a.

Particularly, the input terminals of N voltage conversion units are connected in parallel and then connected to the total input terminal of the high-voltage generating circuit, and the output terminals of N voltage conversion units are connected in series and then connected to the total output terminal of the high-voltage generating circuit. The N energy storage units correspond one-to-one to the N voltage conversion units, and each of the energy storage units is connected between the output terminals of the corresponding voltage conversion units. Each of the N voltage conversion units is used to perform voltage conversion on the input voltage of the total input terminal and charge the corresponding energy storage unit. The input terminal of the voltage adjustment unit 200a is connected to the output terminal of each voltage conversion unit, and the output terminal of the voltage adjustment unit 200a is connected to the total output terminal so as to control the on-off switching between the output terminal of each voltage conversion unit and the total output terminal. The control unit 300a is connected to the N voltage conversion units and the voltage adjustment unit 200a so as to obtain a target voltage of the high-voltage generating circuit, and the N voltage conversion units and the voltage adjustment unit 200a are controlled based on the target voltage so as to adjust the output voltage of the total output terminal to be a target voltage.

Particularly, the voltage conversion units and energy storage units may comprise multiple units, and the specific number may be selected according to actual needs of practice. As a particular example, referring to Fig. 1, the high-voltage generating circuit may comprise four voltage conversion units (voltage conversion units 111a, 121a, 131a, and 141a respectively), and four energy storage units (energy storage units 112a, 122a, 132a and 142a respectively). Particularly, the first input terminals of the voltage conversion units 111a, 121a, 131a and 141a are connected and then connected to a total input positive terminal Vin of the high-voltage generating circuit; the second input terminals of the voltage conversion units 111a, 121a, 131a and 141a are connected and then connected to a total input negative terminal GND of the high-voltage generating circuit. A first output terminal of the voltage conversion unit 111a is connected to one end of the corresponding energy storage unit 112a and a first input terminal of the voltage adjustment unit 200a, and a second output terminal of the voltage conversion unit 111a is connected to the other end of the corresponding energy storage unit 112a. A first output terminal of the voltage conversion unit 121a is connected to one end of the corresponding energy storage unit 122a, a second input terminal of the voltage adjustment unit 200a, and the other end of the energy storage unit 112a. A second output terminal of the voltage conversion unit 121a is connected to the other end of the corresponding energy storage unit 122a. A first output terminal of the voltage conversion unit 131a is connected to one end of the corresponding energy storage unit 132a, a third input terminal of the voltage adjustment unit 200a, and the other end of the energy storage unit 122a. A second output terminal of the voltage conversion unit 131a is connected to the other end of the corresponding energy storage unit 132a. A first output terminal of the voltage conversion unit 141a is connected to one end of the corresponding energy storage unit 142a, a fourth input terminal of the voltage adjustment unit 200, and the other end of the energy storage unit 132a. A second output terminal of the voltage conversion unit 141a is connected to the other end of the corresponding energy storage unit 142a and the total output negative terminal GND of the high-voltage generating circuit. The output terminal of the voltage adjustment unit 200a is connected to the total output positive terminal Vout of the high-voltage generating circuit. The voltage conversion units 111a, 121a, 131a and 141a respectively perform voltage conversion on the input voltage at the total input terminal so as to charge the corresponding energy storage units 112a, 122a, 132a and 142a. When the high-voltage generating circuit is operating, although the input terminals of the voltage conversion units 111a, 121a, 131a and 141a are connected in parallel and the same operating voltage can be obtained, because the output terminals of the voltage conversion units 111a, 121a, 131a and 141a are connected in series, the voltages at the output terminals of the voltage conversion units 141a, 131a, 121a and 111a will increase step by step, i.e., the voltages at one end of the energy storage units 142a, 132a, 122a and 112a will increase step by step. That is to say, the voltages at points D, C, B and A in Fig. 1 increase sequentially, for example, the voltage at point D is the voltage of the energy storage unit 142a; the voltage at point C is the sum of the voltages of the energy storage units 142a and 132a; the voltage at point B is the sum of the voltages of the energy storage units 142a, 132a and 122a; and the voltage at point A is the sum of the voltages of energy storage units 142a, 132a, 122a and 112a.

The control unit 300a obtains a target voltage of the high-voltage generating circuit, and controls the voltage conversion units 111a, 121a, 131a, 141a and the voltage adjustment unit 200a based on the target voltage so as to quickly adjust the output voltage of the total output terminal to be a target voltage. For example, assuming that the maximum voltage corresponding to each energy storage unit is 500V, the voltage can be adjusted in the range of 0-2000V. When a target voltage of 2000V is required, four voltage conversion units can be controlled to work simultaneously to charge the corresponding energy storage units, until the target voltage of 2000V is reached; compared with using only one voltage conversion unit, the acquisition speed of the output voltage is effectively improved. When a target voltage is switched from 2000V to 500V, four energy storage units can be discharged at the same time; compared with using only one energy storage unit, the discharge time is shorter, so the output voltage can be switched quickly. Alternatively, the control unit 300a directly controls the voltage adjustment unit 200a, rapid switching of the output voltage of the total output terminal is achieved by controlling the on-off switching between the first output terminals of the voltage conversion units 111a, 121a, 131a and 141a and the total output positive terminal Vout. For example, the control unit 300a directly controls the output terminal of the voltage conversion unit 141a to be connected to the total output positive terminal Vout, so that the output voltage of the output terminal directly reaches a target voltage of 500V without the need for discharge, thereby achieving rapid switching of the output voltage, and saving electric energy. Moreover, a wide voltage range of, for example 0-2000V, can be achieved, but a solution with only one voltage conversion unit either cannot achieve a voltage range of 0-2000V, or even if it can be achieved, it requires a large transformer and high-power transistor. However, this application can not only achieve a wide range of voltage output, but also because the output voltage is divided into N parts, which is equivalent to low-voltage control, each voltage conversion unit can be implemented with smaller components.

As a result, the output voltage can be quickly switched by using a plurality of input-parallel and output-series voltage conversion units, and the output range of the voltage is widened, thereby improving the application prospects of pulsed electric field ablation technology in arrhythmia treatment.

In an implementation of the first embodiment, N voltage conversion units cooperate with N energy storage units to form N voltage intervals. The control unit 300a is specifically used for: obtaining a voltage interval corresponding to the target voltage, and the N voltage conversion units and the voltage adjustment unit 200a are controlled based on the voltage interval corresponding to the target voltage.

Particularly, continuing to refer to Fig. 1, four voltage conversion units can cooperate with four energy storage units to form four voltage intervals. Assuming that the highest voltages at points A, B, C and D are 2000V, 1500V, 1000V, and 500V respectively, the four voltage intervals are formed as [0V, 500V], [500V, 1000V], [1000V, 1500V], and [1500V, 2000V].

Assuming that a target voltage is switched from 2000V to 500V, then the target voltage obtained by the control unit 300a is 500V, and the corresponding voltage interval is [0V, 500V]. At this time, the control unit 300a can directly control the voltage adjustment unit 200a so that the output of the voltage conversion unit 141a terminal is connected to the total output terminal Vout, while the output terminals of other voltage conversion units are disconnected from the total output positive terminal Vout. At this time, a voltage of 500V can be obtained at the total output terminal. Compared with a solution with only one voltage conversion unit and one energy storage unit, there is no need to reduce the voltage from 2000V to 500V before outputting, thereby greatly improving the switching speed of a output voltage.

For another example, assuming that a target voltage is switched from 2000V to 700V, then the target voltage obtained by the control unit 300a is 700V, and its corresponding voltage interval is [500V, 1000V]. At this time, the control unit 300a can control the voltage conversion units 141a and 131a to charge the corresponding energy storage unit until the voltage at point C reaches 700V, and then the voltage adjustment unit 200a is controlled so that the output terminal of the voltage conversion unit 131a is connected to the total output terminal Vout, while the output terminals of other voltage conversion units are disconnected from the total output positive terminal Vout; at this time, a voltage of 700V can be obtained at the total output terminal. Alternatively, the control unit 300a controls the voltage conversion units 141a and 131a to charge the corresponding energy storage unit so that the voltage at point C reaches 1000V, and then the energy storage unit 132a discharges 300V until the voltage at point C is 700V. Finally, the voltage adjustment unit 200a is controlled so that the output terminal of the voltage conversion unit 131a is connected to the total output terminal Vout, while the output terminals of other voltage conversion units are disconnected from the total output positive terminal Vout; at this time, a voltage of 700V is obtained at the total output terminal, compared with the solution having only one voltage conversion unit and one energy storage unit, there is no need to reduce the voltage from 2000V to 700V before outputting, thereby greatly improving the switching speed of a output voltage.

It should be noted that, this is only an illustrative explanation. In actual applications, other switching manners (such as MOSFET\IGBT and other transistors) can also be used. The details can be determined based on the voltages of each energy storage unit before switching, the target voltage after switching, and the voltage range of the target voltage.

For example, assuming that a target voltage is first switched from 2000V to 300V, then the target voltage obtained by the control unit 300a is 300V, and the corresponding voltage range is [0V, 500V]. At this time, the control unit 300a firstly controls the energy storage unit 142a to discharge so that the voltage at both ends is discharged from 500V to 300V, and then the voltage adjustment unit 200a is controlled so that the output terminal of the voltage conversion unit 141a is connected to the total output positive terminal Vout, while the output terminals of other voltage conversion units are disconnected from the total output positive terminal Vout; at this time, a voltage of 300V can be obtained at the total output terminal, compared with a solution with only one voltage conversion unit and one energy storage unit, there is no need to reduce the voltage from 2000V to 300V before outputting, thereby greatly improving the switching speed of the output voltage.

Further, assuming that a target voltage is switched from 300V to 700V, then the target voltage obtained by the control unit 300a is 700V, and the corresponding voltage range is [500V, 1000V]. At this time, since the 500V previously stored in the energy storage unit 132a has not been discharged, the voltage at point C is 800V. Now the energy storage unit 142a and/or the energy storage unit 132a can be discharged so that the voltage at point C is 700V. Then the control unit 300a controls the voltage adjustment unit 200a so that the output terminal of the voltage conversion unit 131a terminal is connected to the total output positive terminal Vout, while the output terminals of other voltage conversion units are disconnected from the total output terminal Vout; at this time, a voltage of 700V can be obtained at the total output terminal, compared with a solution with only one voltage conversion unit and one energy storage unit, there is no need to charge from 300V to 700V again, which not only has a faster switching speed of the output voltage, but also saves energy.

Further, assuming that a target voltage is switched from 700V to 1300V, the target voltage obtained by the control unit 300a is 1300V, and the corresponding voltage interval is [1000V, 1500V]. At this time, since the 500V previously stored in the energy storage unit 122a has not been discharged, the voltage at point B is 1200V. Now the voltage conversion unit 141a and/or 131a are controlled to recharge by 100V to obtain a voltage of 1300V at point B. Compared with a solution with only one voltage conversion unit and one energy storage unit, there is no need to charge from 700V to 1300V, which not only has a faster switching speed of the output voltage, but also saves energy. For other situations, we will not list them one by one herein. Therefore, by obtaining the voltages of each energy storage unit before the target voltage is switched, multiple voltage conversion units are controlled based on the voltage of each energy storage unit and the target voltage, and the voltage adjustment unit is controlled based on a voltage range corresponding to the target voltage, thereby enabling rapid switching of the output voltage.

In an implementation of the first embodiment, as shown in Fig. 2, the high-voltage generating circuit for a catheter further comprises: a current adjustment unit 400a, whose input terminal is connected to an output terminal of a voltage adjustment unit 200a, wherein an output terminal of the current adjustment unit 400a is connected to the total output positive terminal Vout so as to adjust the output current of the total output positive terminal Vout; a control unit 300a is also connected to the current adjustment unit 400a so as to obtain a target power of the high-voltage generating circuit; the N voltage conversion units, the voltage adjustment unit 200a and the current adjustment unit 400a are controlled based on the target power of the high-voltage generating circuit, so that the output voltage and output current of the total output positive terminal Vout are adjusted to obtain the target power.

Particularly, the control unit 300a can control the current adjustment unit 400a to change the output current of the total output terminal by changing the size of the built-in resistor of the current adjustment unit 400a. For example, the current adjustment unit 400a can be a variable resistor whose resistance can be infinitely adjusted, by changing the resistance of the variable resistor, the output current can be directly adjusted to the required current, thereby achieving rapid switching of different output currents. Further, the control unit 300a can realize flexible adjustment of the output voltage, output current and output power by controlling the N voltage conversion units, the voltage adjustment unit 200a and the current adjustment unit 400a. Particularly, when only a target power is required, the output voltage and/or output current of the total output terminal can be adjusted through the aforementioned method so that the output power reaches the target power; when there are requirements for both target power and target voltage, the output voltage of the total output terminal can be adjusted through the aforementioned method to reach the target voltage, and then the output current can be changed by adjusting the internal resistance of the current adjustment unit 400a, so that the output power reaches the target power.

It should be noted that, the control unit 300a can also control the N voltage conversion units, the voltage adjustment unit 200a and the current adjustment unit 400a to realize rapid switching of a target power. For example, when only a target power is required, assuming that the target power is 1000w, and the target voltage before switching is 800V, if the output current can reach 1.25A, then rapid adjustment of the target power can be achieved by adjusting the output current. Compared with the solution with only one voltage conversion unit and a corresponding energy storage unit, the process of output voltage adjustment is eliminated, and the adjustment of the output voltage involves the charge and discharge process, the adjustment speed of a target power may be faster through the current adjustment unit 400a, and the adjustment is more energy efficient. When there are requirements for both target power and target voltage, this embodiment can achieve the output of any target voltage and any target power within the above range, and there may be no constraint relationship between the target power and the target voltage. For example, as for a solution with only one voltage conversion unit and a corresponding energy storage unit, the target power and target voltage are in a corresponding relationship and cannot be adjusted flexibly.

As a result, multiple voltage conversion units, voltage adjustment units, and current adjustment units can be used to realize fast and flexible switching of different output voltages, output currents, and output powers. At the same time, because the output voltage is divided into N parts, it is equivalent to low-voltage control, so that each voltage conversion unit can be implemented by using smaller components, thereby improving the application prospects of pulsed electric field ablation technology in arrhythmia treatment.

In an implementation of the first embodiment, the control unit 300a comprises one or more control chips, when the control unit 300a comprises one control chip, the N voltage conversion units share the control chip; or when the control unit 300a comprises multiple control chips, some of the N voltage conversion units share one control chip, or each voltage conversion unit corresponds to one of the control chips.

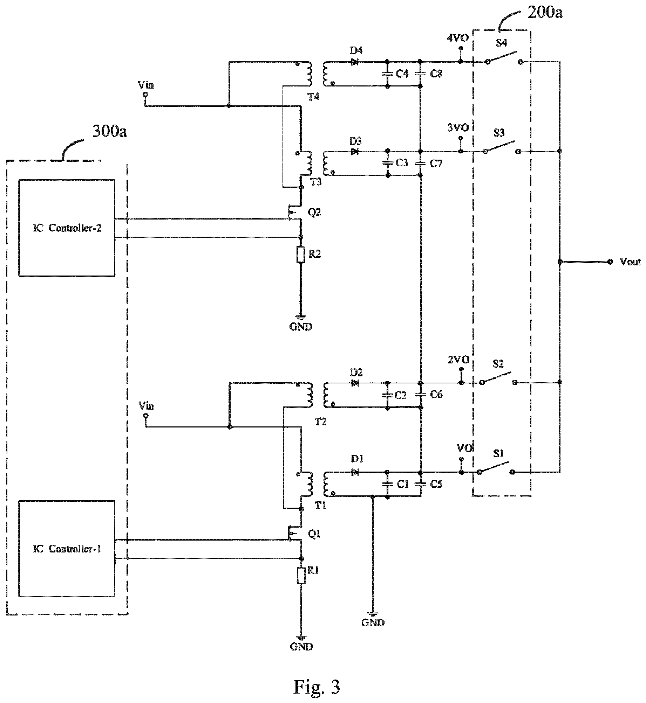

Particularly, the control chip in the control unit 300a may comprise one or more control chips. Each control chip may control one voltage conversion unit or multiple voltage conversion units. The specific number of control chips and the number of voltage conversion units controlled by each control chip can be selected according to actual needs of practice. As a particular example, as shown in Fig. 3, the control unit 300a of the high-voltage generating circuit comprises two control chips, namely the control chip IC Controller-1 and the control chip IC Controller-2, and each control chip simultaneously controls two voltage conversion units, the same control chip has the same switching cycle, so a voltage conversion unit controlled by the same control chip can output a conversion voltage more flexibly, and by controlling the time period of the on-off switching of different control chips, staggered switching of different control chips can be achieved to realize the minimum input current ripple and minimum voltage ripple, thereby inputting the minimum capacitance.

In an implementation of the first embodiment, the N voltage conversion units have the same structure, each voltage conversion unit comprises a switch tube for voltage conversion, and some or all of the N voltage conversion units share a switch tube.

That is to say, N voltage conversion units can share one or more switch tubes to perform voltage conversion through one or more switch tubes. The specific number of switch tubes and the number of voltage conversion units controlled by each switch tube can be selected according to actual usage needs. As a particular example, continuing to refer to Fig. 3, the high-voltage generating circuit comprises two switch tubes, and each switch tube simultaneously controls two voltage conversion units. When the control unit 300a controls the on-off switching of a switch tube, it can control the connection or disconnection to a voltage conversion unit corresponding to the switch tube, thereby saving the cost and layout size.

In an implementation of the first embodiment, each voltage conversion unit is a flyback conversion circuit, a forward conversion circuit, an LLC resonance circuit, a push-pull circuit or a bridge circuit. That is to say, the voltage conversion unit in this application can be a flyback conversion circuit, a forward conversion circuit, an LLC resonance circuit, a push-pull circuit or a bridge circuit, etc.; the type of the voltage conversion units must be consistent during use, and the specific method can be selected and set according to actual needs.

In an implementation of the first embodiment, as shown in Fig. 3, the flyback conversion circuit comprises: a transformer (such as T1), a first switch tube (such as Q1), a first diode (such as D1) and a filter capacitor (such as C1), wherein one end of the primary winding of the transformer (such as T1) is connected to the total input positive terminal Vin; a first end of the first switch tube (such as Q1) is connected to the other end of the primary winding of the transformer (such as T1), a second end of the first switch tube (such as Q1) is connected to the ground GND, the control terminal of the first switch tube (such as Q1) is connected to the control unit 300a; the anode of the first diode (such as D1) is connected to one end of the secondary winding of the transformer (such as T1); the filter capacitor (such as C1) is connected in parallel between the output terminals of the corresponding voltage conversion units, one end of the filter capacitor (such as C1) is connected to the cathode of the first diode (such as D1), and the other end of the filter capacitor (such as C1) is connected to the other end of a secondary winding of the transformer (such as T1).

Optionally, a first resistor (such as R1) is connected in series between a second end of the first switch tube (such as Q1) and the ground, and the connection point between the first resistor (such as R1) and a second end of the first switch tube (such as Q1) is connected to the control unit 300a.

Optionally, the N energy storage units have the same structure, and each energy storage unit comprises an energy storage capacitor (such as C5) which is connected in parallel between the output terminals of the corresponding voltage conversion units.

The following description takes Fig. 3 as an example, in which each voltage conversion circuit is a flyback conversion circuit. In Fig. 3, the control unit 300a comprises two control chips, each control chip controls two flyback conversion circuits at the same time, and every two flyback conversion circuits share a switch tube.

Particularly, as shown in Fig. 3, a first flyback conversion circuit comprises: a transformer T1, a first switch tube Q1, a first diode D1 and a filter capacitor C1; and a second flyback conversion circuit comprises: a transformer T2, a first switch tube Q2, first diode D2 and a filter capacitor C2; a third flyback conversion circuit comprises: a transformer T3, a first switch tube Q3, a first diode D3 and a filter capacitor C3; and a fourth flyback conversion circuit comprises: a transformer T4 , a first switch tube Q4, a first diode D4 and a filter capacitor C4. Particularly, one end of the primary windings of the transformers T1, T2, T3 and T4 is connected to the total input positive terminal Vin, a first end of the first switch tube Q1 is connected to the other end of the primary windings of the transformers T1 and T2, and a first end of the first switch tube Q2 is connected to the other end of the primary windings of the transformers T3 and T4; the second ends of the first switch tubes Q1 and Q2 are connected to ground, and the control terminal of the first switch tube Q1 is connected to the control chip IC Controller-1, and the control terminal of the first switch tube Q2 is connected to the control chip IC Controller-2. The anodes of the first diodes D1, D2, D3 and D4 are respectively connected to one end of the secondary windings of the transformers T1, T2, T3 and T4. The filter capacitor C1, C2, C3 and C4 are connected in parallel between the output terminals of the corresponding voltage conversion units; one end of the filter capacitors C1, C2, C3 and C4 is respectively connected to the cathode of the first diodes D1, D2, D3 and D4; the other end of the filter capacitors C1, C2, C3 and C4 are respectively connected to the other end of the secondary windings of the transformers T1, T2, T3 and T4; and the secondary winding of the transformer T1 is connected to ground. A first resistor R1 is also connected in series between a second end of the first switch tube Q1 and the ground. The connection point between the first resistor R1 and a second end of the first switch tube Q1 is connected to the control chip IC Controller-1. A first resistor R2 is also connected in series between a second end of the switch tube Q2 and the ground. The connection point between the first resistor R2 and a second end of the first switch tube Q2 is connected to the control chip IC Controller-2. Each energy storage unit comprises an energy storage capacitor, and the energy storage capacitors C5, C6, C7 and C8 are respectively connected in parallel between the output terminals of the corresponding flyback conversion circuits.

When the high-voltage generating circuit is operating, the primary windings of the transformers T1, T2, T3 and T4 obtain the same operating voltage from the total input positive terminal Vin. When the control unit 300a controls the first switch tubes Q1 and Q2 to turn on, the current in the primary windings of the transformers T1, T2, T3 and T4 and the magnetic field in the core increase, so as to store energy in the core. Since the voltage generated in the secondary windings of the transformers T1, T2, T3 and T4 is reversed, so that the first diodes D1, D2, D3 and D4 are in a reverse biased state and cannot be connected; at this time, the energy storage capacitors C5, C6, C7 and C8 provide voltage and current to the load. When the control unit 300a controls the first switch tubes Q1 and Q2 to turn off, the current in the primary winding is 0, and at the same time the magnetic field in the core begins to decrease, and a forward voltage is induced on the secondary winding; at this time, the first diodes D1, D2, D3 and D4 are in a positive biased state, the conducting current flows into the energy storage capacitors C5, C6, C7, C8 and the load, and the energy stored in the magnetic core is transferred to the energy storage capacitors C5, C6, C7, C8 and the load. Since the secondary winding of transformer T1 is connected to ground, the output voltage of the first flyback conversion circuit is the absolute voltage VO, and so on, the output voltage of the second flyback conversion circuit is 2VO, and the output voltage of the third flyback conversion circuit is 3VO, the output voltage of the fourth flyback conversion circuit is 4VO, thereby achieving a step-by-step increase in the output voltage.

In an implementation of the first embodiment, the voltage adjustment unit 200a comprises: N first switches which correspond one-to-one to the N voltage conversion units, and each first switch is connected in series between an output terminal of the corresponding voltage conversion unit and the total output positive terminal Vout.

Particularly, continuing to refer to Fig. 3, the voltage adjustment unit 200a comprises four first switches, namely first switches S1, S2, S3 and S4. The first switches S1, S2, S3 and S4 are respectively connected in series between the output terminals of the corresponding voltage conversion units and the current adjustment unit 400. By controlling the on-off switching of the first switches S1, S2, S3 and S4, the voltage output of the corresponding voltage conversion unit is realized, thereby realizing rapid switching of the output voltage. For example, when the first switch S4 is turned on and the other first switches are turned off, the output voltage is VO; alternatively, when the first switch S1 is turned on and the other first switches are turned off, the output voltage is 4VO, thereby realizing the rapid switching of the output voltage from VO to 4VO, and expanding the output voltage range.

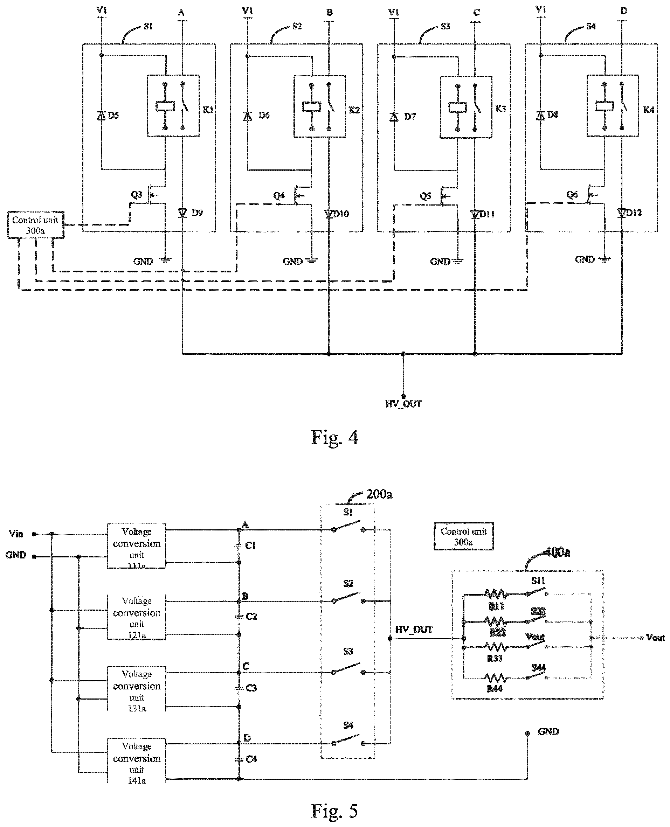

Further, as shown in Fig. 4, the N first switches have the same structure, and each first switch comprises: a relay (such as K1), a voltage stabilizing tube (such as D5), a second diode (such as D9), and a second switch tube (such as Q3); wherein one end of the switch of the relay (such as K1) is connected to an output terminal of the corresponding voltage conversion unit, and one end of the coil of the relay (such as K1) is connected to the preset power supply V1; the anode of the voltage stabilizing tube (such as D5) is connected to the other end of the coil of the relay (such as K1), and the cathode of the voltage stabilizing tube (such as D5) is connected to said one end of the coil of the relay (such as K1); the anode of the second diode (such as D9) is connected to the other end of the switch of the relay (such as K1), and the cathode of the second diode (such as D9) is connected to the current adjustment unit 400a; the first end of the second switch tube (such as Q3) is connected to the other end of the coil the relay (such as K1), and a second end of the second switch tube (such as Q3) is connected to the ground, and the control terminal of the second switch tube (such as Q3) is connected to the control unit 300.

Particularly, the following description takes Fig. 4 as an example to illustrate four first switches respectively corresponding to four voltage conversion units. Referring to Fig. 4, the first switch S1 comprises: a relay K1, a voltage stabilizing tube D5, a second diode D9 and a second switch tube Q3. The first switch S2 comprise: a relay K2, a voltage stabilizing tube D6, a second diode D10 and a second switch tube Q4. The first switch S3 comprises: a relay K3, a voltage stabilizing tube D7, a second diode D11 and a second switch tube Q5. The first switch S4 comprises: a relay K4, a voltage stabilizing tube D8, a second diode D12 and a second switch tube Q6. Particularly, one end of the switches of relays K1, K2, K3 and K4 is respectively connected to the output terminal of the corresponding voltage conversion units; one end of the coils of relays K1, K2, K3 and K4 is connected to the preset power supply V1. The anodes of the voltage stabilizing tubes D5, D6, D7 and D8 are respectively connected to the other end of the coils of relays K1, K2, K3 and K4; and the cathodes of the voltage stabilizing tubes D5, D6, D7 and D8 are respectively connected to one end of the coils of relays K1, K2, K3 and K4. The anodes of the second diodes D9, D10, D11 and D12 are respectively connected to the other end of the switches of the relays K1, K2, K3 and K4; and the cathodes of the second diodes D9, D10, D11 and D12 are respectively connected to the current adjustment Unit 400. A first end of the second switch tubes Q3, Q4, Q5 and Q6 are respectively connected to the other end of the coils of the relays K1, K2, K3 and K4, and a second end of the second switch tubes Q3, Q4, Q5 and Q6 are all connected to ground, and the control terminals of the second switch tubes Q3, Q4, Q5 and Q6 are respectively connected to the control unit 300.

When the high-voltage generating circuit is operating, by controlling the on-off switching of relays K1, K2, K3 and K4, the final output voltage of the total output positive terminal Vout can be controlled. For example, when the control unit 300 controls the second switch Q3 to turn on, and controls the remaining second switch tubes to turn off, that is to say, the relay K1 is controlled to be turned on, and the other relays are turned off, the output voltage of the total output positive terminal Vout is VO. When the control unit 300 controls the second switch tube Q4 to turn on, and controls the remaining second switch tubes to turn off, that is to say, the relay K2 is controlled to be turned on, and the other relays are turned off, the output voltage of the total output positive terminal Vout is 2VO, ..., and so on, thereby achieving a step-by-step increase and rapid switching of the output voltage.

It should be noted that, the second switch tubes Q3, Q4, Q5 and Q6 can prevent the voltage backflow caused by accidental switching-on of an upper-stage relay. Even if the relays K1, K2, K3 and K4 are all turned on, the high voltage 4VO correspondingly output by the relay K1 will not flow back into other low-voltage circuits and cause components to burn out; and if a subsequent stage relay does not work, for example, relative to relay K2, relay K1 does not work, then relay K1 is equivalent to no load, and the back end of relay K1 is equivalent to floating, so there will be no voltage difference problem, that is to say, a low-voltage relay can continue to be used.

In an implementation of the first embodiment, as shown in Fig. 5, the current adjustment unit 400a comprises M current adjustment branches connected in parallel, and each current adjustment branch comprises: a current limiting resistor (such as R11), one end of which is connected to an output terminal of the voltage adjustment unit 200a; and a current-limiting switch (such as S11), one end of which is connected to the other end of the current-limiting resistor (such as R11), wherein the other end of the current-limiting switch (such as S11) is connected to the total output positive terminal Vout.

Particularly, four current adjustment branches are taken as an example. The first current adjustment branch comprises: a current limiting resistor R11, and a current limiting switch S11; wherein one end of the current limiting resistor R11 is connected to the output terminal of the voltage adjustment unit 200a, one end of the current-limiting switch S11 is connected to the other end of the current-limiting resistor R11, and the other end of the current-limiting switch S11 is connected to the total output positive terminal Vout. The second current adjustment branch comprises: a current-limiting resistor R22, and a current-limiting switch S22; wherein one end of the current-limiting resistor R22 is connected to the output terminal of the voltage adjustment unit 200, one end of the current-limiting switch S22 is connected to the other end of the current-limiting resistor R22, and the other end of the current-limiting switch S22 is connected to the total output positive terminal Vout. The third current adjustment branch comprises: a current-limiting resistor R33, and a current-limiting switch S33; wherein one end of the current-limiting resistor R33 is connected to the output terminal of the voltage adjustment unit 200, one end of the current-limiting switch S33 is connected to the other end of the current-limiting resistor R33, and the other end of the current-limiting switch S33 is connected to the total output positive terminal Vout. The fourth current adjustment branch comprises: a current-limiting resistor R44, and a current-limiting switch S44; wherein one end of the current-limiting resistor R44 is connected to the output terminal of the voltage adjustment unit 200a, one end of the current-limiting switch S44 is connected to the other end of the resistor R44, and the other end of the current limiting switch S44 is connected to the total output positive terminal Vout. It should be noted that, the resistance values of the current limiting resistors R11, R22, R33 and R44 can be the same or different.

When the high-voltage generating circuit is operating, as shown in Fig. 5, the first to fourth current adjustment branches are connected in parallel, and the voltage obtained by each current adjustment branch is HV_OUT. The on-off status of different current adjustment branches are controlled by controlling the on-off switching of the current limiting switch in the current adjustment branch, so that the output current of the total output positive terminal Vout can be adjusted based on the current-limiting resistance of the conducting current adjustment branch. For example, when the voltage HV_OUT input to the current adjustment unit 400a remains unchanged, the current-limiting switch S11 is turned on, and the other current-limiting switches are turned off, then the output current is the ratio of the input voltage HV_OUT to the current-limiting resistor R11 plus the load resistance; when the current-limiting switches S11 and S22 are turned on, and the other current-limiting switches are turned off, then the output current is the ratio of the input voltage HV_OUT to the current-limiting resistors R11 and R22 in parallel plus the load resistance. Thus, it is possible to realize fast switching of output current, that is to say, by selecting current-limiting resistors with different resistance values, or selecting different numbers of current-limiting resistors, the output current of the total output positive terminal Vout can be adjusted.

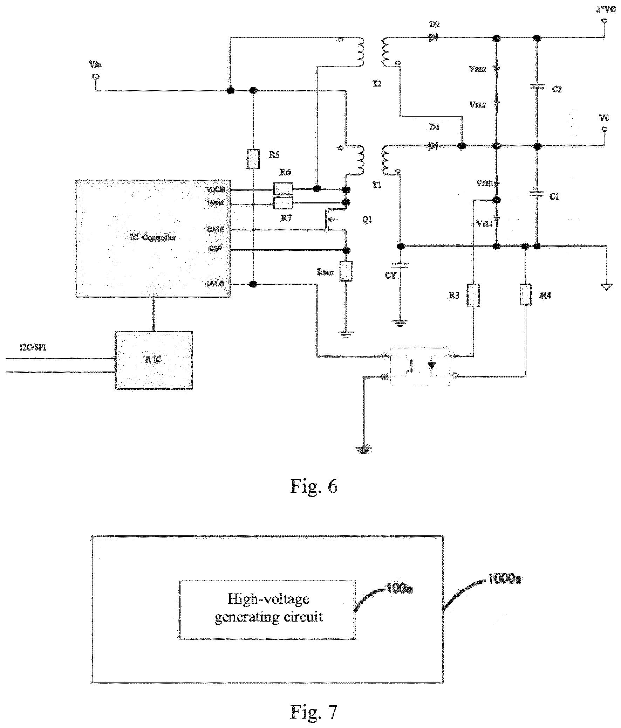

In an implementation of the first embodiment, as shown in Fig. 6, the high-voltage generating circuit for a catheter further comprises: an overvoltage protection unit, which comprises voltage stabilizing tubes (such as VzH1 and VzL1, as well as VzH2 and VzL2) connected in parallel with each of the energy storage units.

When the voltage is too high, for example, when VO or 2VO is too high, a voltage stabilizing tube will be reversely broken down, so that a voltage across the terminals (the voltage across VzH1 and VzL1, as well as the voltage across VzH2 and VzL2) will almost remain unchanged and show voltage stabilizing characteristics, thereby playing a role of overvoltage protection, preventing the output voltage from being too high, and preventing electronic components (such as output capacitors, diodes, input MOSFETs, etc.) from being damaged by overvoltage.

Preferably, there are more than two voltage stabilizing tubes connected in parallel with each energy storage unit, so that a more appropriate overvoltage protection range can be achieved by combining two or more voltage stabilizing tubes.

Further, as shown in Fig. 6, the overvoltage protection unit also comprises an optical coupler, and each of the optical couplers corresponds to a control chip; wherein in each voltage conversion unit controlled by the control chip and the corresponding energy storage unit, at least two or more voltage stabilizing tubes are connected in parallel to at least an energy storage unit closest to the low voltage end, a light emitter of the optical coupler is connected in parallel with part of the voltage stabilizing tubes (i.e., not connected in parallel with all the voltage stabilizing tubes, as shown in Fig. 6, when there are two voltage stabilizing tubes VzH1 and VzL1, only VzL1 is connected in parallel), and an output terminal of the light receptor of the optical coupler is connected to the under-voltage protection (UVLO) pin of the control chip.

It should be pointed out that, the circuit diagram shown in Fig. 6 is only a schematic diagram of a circuit controlled by one control chip in which an overvoltage protection unit is arranged. When multiple control chips are provided (as shown in Fig. 2, two control chips IC Controller-1 and IC Controller-2 are provided), those skilled in the art know to arrange a corresponding overvoltage protection unit in each circuit controlled by a control chip.

When only the voltage stabilizing tube is arranged for voltage stabilization protection, although it can play a certain overvoltage protection role, the breakdown of the voltage stabilizing tube (such as VzH1 and VzL1, as well as VzH2 and VzL2) by long-term overvoltage is apt to cause overheating of the voltage stabilizing tube and burn it out, resulting in the inability to stabilize the voltage.

In this embodiment, there are two or more voltage stabilizing tubes (particularly two, namely VzH1 and VzL1) connected in parallel to at least an energy storage unit (particularly C1) closest to the low voltage end, therefore, when VO is too high, the voltage stabilizing tubes VzH1 and VzL1 are reversely broken down, so that a voltage exists across both ends of VzL1, so that the light emitter of the optical coupler emits light, the light receptor of the optical coupler outputs a signal to the control chip (IC controller), and the control chip (IC controller) does not work, or the control chip (IC controller) reduces the duty ratio, causing the subsequent output voltage to decrease, thereby safely controlling the output overvoltage and not causing long-term overvoltage and damaging to the voltage stabilizing tube, thereby achieving safer overvoltage protection.

In addition, when outputting in high-voltage superposition mode (such as 2*VO), any output overvoltage controlled by a control chip can be balanced by the voltage stabilizing tube, and then output to the lowest stage and fed back to the control chip (IC controller), so that the maximum values of each output voltage are balanced and limited. To sum up, according to the high-voltage generating circuit for a catheter according to the embodiment of the present application, the input terminals of the voltage conversion units are connected in parallel, and then connected to the total input terminal of the high-voltage generating circuit; and the output terminals of the voltage conversion units are connected in series, and then connected to a total output terminal of the high-voltage generating circuit. The voltage adjustment unit is connected between the output terminal of each voltage conversion unit and the total output terminal so as to control the on-off switching between the output terminal of each voltage conversion unit and the total output terminal. The control unit is connected to each voltage conversion unit and the voltage adjustment unit so as to control the voltage conversion unit and the voltage adjustment unit to adjust the output voltage of the total output terminal to be a target voltage. As a result, the output voltage can be quickly switched to the target voltage by using a plurality of input-parallel and output-series voltage conversion units, and meanwhile, the output range of the voltage is widened. Furthermore, the overvoltage protection unit is used to prevent the output voltage from being too high, and prevent electronic components from being damaged by overvoltage, thereby enhancing the safety of pulsed electric field ablation technology and improving its application prospects in arrhythmia treatment.

Fig. 7 is a schematic structural diagram of an ablation tool according to a second embodiment of the present application. Referring to Fig. 7, the ablation tool 1000a comprises the above-mentioned high-voltage generating circuit 100a for a catheter.

According to the ablation tool of the embodiment of the present application, through the above-mentioned high-voltage generating circuit for a catheter, the output voltage can be quickly switched to a target voltage by using a plurality of input-parallel and output-series voltage conversion units, and meanwhile, the output range of the voltage is widened, furthermore, the overvoltage protection unit can prevent the output voltage from being too high and prevent overvoltage damage to electronic components, thereby improving the application prospects of pulsed electric field ablation technology in arrhythmia treatment.

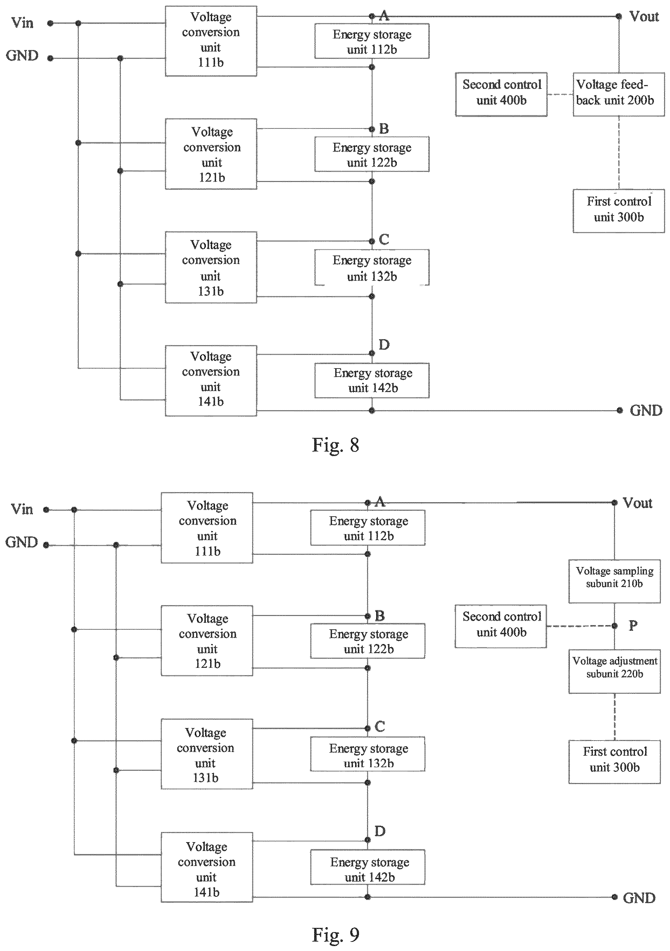

Fig. 8 is a schematic structural diagram of a high-voltage generating circuit for a catheter according to the third embodiment of the present application. Referring to Fig. 8, the high-voltage generating circuit may comprise: N voltage conversion units (N is an integer greater than 1), N energy storage units, voltage feedback unit 200b, a first control unit 300b and a second control unit 400b.

Particularly, the input terminals of N voltage conversion units are connected in parallel and then connected to the total input terminal of the high-voltage generating circuit; the output terminals of N voltage conversion units are connected in series and then connected to the total output terminal of the high-voltage generating circuit; N energy storage units correspond one-to-one to the N voltage conversion units, and each energy storage unit is connected between the output terminals of the corresponding voltage conversion units. Each of the N voltage conversion units is used to perform voltage conversion on the input voltage of the total input terminal and charge the corresponding energy storage unit; the detection terminal of the voltage feedback unit 200b is connected to the total output terminal used to detect the output voltage of the total output terminal to obtain the feedback voltage and adjust the feedback voltage. A first control unit 300b is connected to the adjustment terminal of the voltage feedback unit 200b so as to control the voltage feedback unit 200b to adjust the feedback voltage; a second control unit 400b is connected to the output terminals of the N voltage conversion units and the voltage feedback unit 200b, used to obtain a target voltage of the high-voltage generating circuit; and the N voltage conversion units are controlled based on the target voltage and the adjusted feedback voltage, so as to adjust the output voltage of the total output terminal to obtain a target voltage.

It should be noted that, the voltage conversion unit, energy storage unit, etc. in this embodiment are arranged in the same manner as those in the first embodiment. Those skilled in the art know that the voltage feedback unit 200b, a first control unit 300b, a second control unit 400b etc. in this embodiment can also be arranged in the high-voltage generating circuit for a catheter of the first embodiment, so as to adjust the output voltage of the total output terminal to obtain a target voltage.

Particularly, the voltage conversion units and energy storage units may comprise multiple units, and the specific number may be selected according to actual needs of practice. As a particular example, referring to Fig. 8, the high-voltage generating circuit may comprise four voltage conversion units (voltage conversion units 111b, 121b, 122b, 131b, and 141b respectively) and four energy storage units (energy storage units 112b, 122b, 132b, and 142b respectively). Particularly, the first input terminals of the voltage conversion units 111b, 121b, 131b and 141b are connected and then connected to a total input positive terminal Vin of the high-voltage generating circuit; and the second input terminals of the voltage conversion units 111b, 121b, 131b and 141b are connected and then connected to the total input negative terminal GND of high-voltage generating circuit. A first output terminal of the voltage conversion unit 111b is connected to one end of the corresponding energy storage unit 112b; and a second output terminal of the voltage conversion unit 111b is connected to the other end of the corresponding energy storage unit 112b. A first output terminal of the voltage conversion unit 121b is connected to one end of the corresponding energy storage unit 122b and the other end of the energy storage unit 112b; and a second output terminal of the voltage conversion unit 121b is connected to the other end of the corresponding energy storage unit 122b. A first output terminal of the voltage conversion unit 131b is connected to one end of the corresponding energy storage unit 132b and the other end of the energy storage unit 122b; and a second output terminal of the voltage conversion unit 131b is connected to the other end of the corresponding energy storage unit 132b. A first output terminal of the voltage conversion unit 141b is connected to one end of the corresponding energy storage unit 142b, the input terminal of the current adjustment unit 600b and the other end of the energy storage unit 132b; and a second output terminal of the voltage conversion unit 141b is connected to the other end of the corresponding energy storage unit 142b and the total output negative terminal GND of the high-voltage generating circuit. When the high-voltage generating circuit is operating, the input terminals of the voltage conversion units 111b, 121b, 131b and 141b are connected in parallel and they can obtain the same operating voltage. However, since the output terminals of the voltage conversion units 111b, 121b, 131b and 141b are connected in series, the voltages at the output terminals of the voltage conversion units 141b, 131b, 121b and 111b will increase step by step, that is to say, the voltages at one end of the energy storage units 142b, 132b, 122b and 112b will increase step by step, i.e., the voltages at points D, C, B and A in Fig. 8 increase in sequence. For example, the voltage at point D is the voltage of the energy storage unit 142b, the voltage at point C is the sum of the voltages of the energy storage units 142b and 132b, the voltage at point B is the sum of the voltages of the energy storage units 142b, 132b and 122b, and the voltage at point A is the sum of the voltages of energy storage units 142b, 132b, 122b and 112b.

The detection terminal of the voltage feedback unit 200b is connected to the total output positive terminal Vout, so as to detect the output voltage of the total output terminal to obtain the feedback voltage. A first control unit 300b is connected to the adjustment terminal of the voltage feedback unit 200b, and the adjustment of the feedback voltage can be realized by controlling the voltage feedback unit 200b, and then the output voltage is adjusted to a speed of the target voltage, under ideal circumstances, infinitely adjustment can be achieved. For example, when the output voltage needs to be quickly adjusted to a target voltage, the voltage feedback unit 200b can be controlled by a first control unit 300b to adjust the feedback voltage, so that there is a relatively large difference between the output voltage corresponding to the adjusted feedback voltage and the target voltage; and then a rapid adjustment of the output voltage can be realized by performing a closed-loop voltage adjustment based on such a large difference to realize. Conversely, the voltage feedback unit 200b can be controlled by a first control unit 300b to adjust the feedback voltage, so that there is a relatively small difference between the output voltage corresponding to the adjusted feedback voltage and the target voltage; and then a slow adjustment of the output voltage can be realized by performing a closed-loop voltage adjustment based on such a small difference.

A second control unit 400b can control multiple voltage conversion units to quickly switch the output voltage of the total output terminal to the target voltage. For example, assuming that the highest voltage corresponding to each energy storage unit is 500V, the voltage adjustment range is 0-2000V. When a target voltage of 2000V needs to be obtained, four voltage conversion units can be controlled to work simultaneously to charge the corresponding energy storage units until the target voltage of 2000V is reached, and such a speed of obtaining the output voltage is effectively increased as compared with using only one voltage conversion unit. When the target voltage is switched from 2000V to 500V, four energy storage units can be discharged at the same time, and such a discharge time is shorter as compared with using only one energy storage unit, so that the output voltage can be switched quickly; at the same time, a wide voltage range such as 0-2000V can be achieved. However, a solution with only one voltage conversion unit either cannot achieve a voltage range such as 0-2000V, or even if it can be achieved, it requires a large volume transformer and high-power transistor. The present application can achieve a wide range of voltage output, moreover, since the output voltage is divided into N parts, which is equivalent to low-voltage control, each voltage conversion unit can be implemented with smaller components.

Further, when a second control unit 400b controls multiple voltage conversion units based on the feedback voltage and the target voltage, the voltage conversion unit that needs to work, the energy storage unit that needs to be charged and discharged, and the charging and discharging speed of the energy storage unit can be determined based on the difference between the feedback voltage and the target voltage, so as to realize rapid switching of the output voltage to the target voltage. For example, when the output voltage needs to be quickly adjusted to a target voltage, a first control unit 300b can control the voltage feedback unit 200b to adjust the feedback voltage, so that there is a large difference between the output voltage corresponding to the adjusted feedback voltage and the target voltage; then the second control unit 400b can determine the current voltage conversion unit that needs to work or the energy storage unit that needs to be charged and discharged based on the target voltage. Assuming that the current target voltage is 2000V, and then all four voltage conversion units need to work together to charge the corresponding energy storage units. At the same time, the second control unit 400b controls the four voltage conversion units to charge the energy storage units based on the large difference, and adjusts the charging speed of the energy storage units, thereby achieving rapid adjustment of the output voltage to the target voltage. On the contrary, when the output voltage needs to be slowly adjusted to the target voltage of 2000V, a first control unit 300b can control the voltage feedback unit 200b to adjust the feedback voltage, so that there is a small difference between the output voltage corresponding to the adjusted feedback voltage and the target voltage. Then a second control unit 400b can control the four voltage conversion units simultaneously or one by one to charge the energy storage units based on the small difference, and adjust the charging speed of the energy storage units to realize a slow adjustment of the output voltage to the target voltage.

As a result, the output voltage can be quickly switched to a target voltage through multiple voltage conversion units and voltage feedback units, thereby improving the application prospects of pulsed electric field ablation technology in arrhythmia treatment.

In an implementation of the third embodiment, as shown in Fig. 8, the voltage feedback unit 200b comprises: a voltage sampling subunit 210b and a voltage adjustment subunit 220b, wherein one end of the voltage sampling subunit 210b is connected to the total output positive terminal Vout; one end of the voltage adjustment subunit 220b is connected to the other end of the voltage sampling subunit 210b and forms a first node P, the first node P is connected to a second control unit 400b, and the other end of the voltage adjustment subunit 220b is connected to a first control unit 300b.

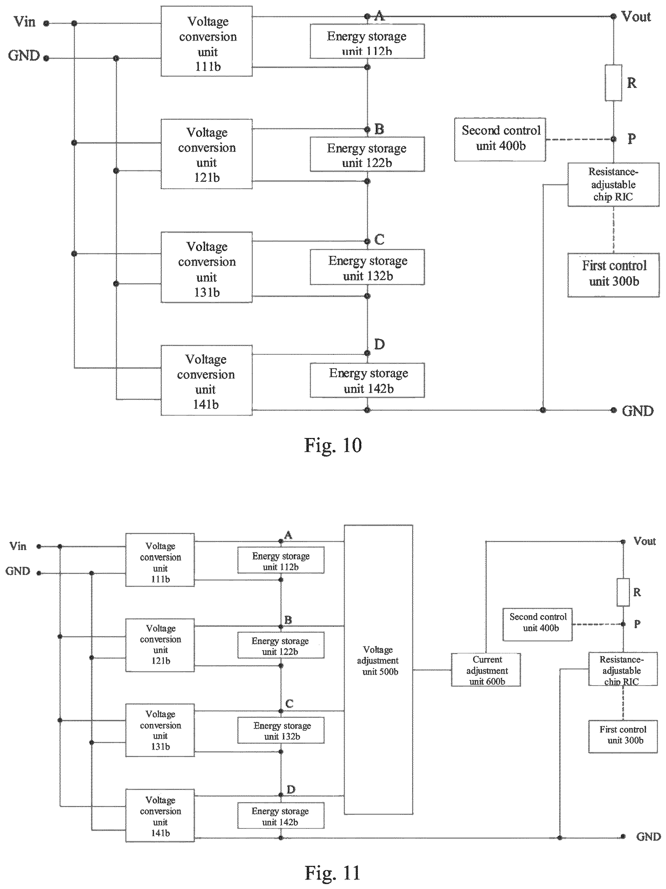

Optionally, as shown in Fig. 10, the voltage sampling subunit 210b comprises: a sampling resistor R, whose one end is connected to the total output positive terminal Vout; the voltage adjustment subunit 220b comprises: a resistance adjustable chip RIC, whose first end is connected to the other end of the sampling resistor R, wherein a second end of the resistance-adjustable chip RIC is connected to ground, and the control terminal of the resistance-adjustable chip RIC is connected to a first control unit 300b. Further, the first control unit 300b is specifically used to send a feedback voltage adjustment instruction to the resistance adjustable chip RIC, so that the resistance adjustable chip RIC obtains the resistance adjustment amount according to the feedback voltage adjustment instruction, and adjusts its own resistance based on the resistance adjustment amount.

Particularly, the sampling resistor R and the resistance-adjustable chip RIC are connected in series to form a voltage sampling circuit so as to collect the output voltage of the total output terminal to obtain the feedback voltage, and send it to a second control unit 400b. A first control unit 300b can generate a feedback voltage adjustment instruction according to the adjustment requirements, and send the voltage adjustment instruction to the resistance-adjustable chip RIC, and the resistance-adjustable chip RIC changes its resistance value according to the instruction so as to change the feedback voltage, thereby allowing the second control unit 400b to quickly adjust the output voltage of the total output positive terminal Vout to be a target voltage based on the target voltage and the adjusted feedback voltage.

For example, assuming that the current output voltage of the total output positive terminal Vout is U1 (i.e., D terminal output), and the sampling resistor R is a fixed value Rx, and the initial resistance of the resistance-adjustable chip RIC is Rx. If it is currently necessary to increase the voltage at the total output positive terminal Vout from U1 to U2, the feedback voltage is U1/2 without changing the resistance value of the resistance-adjustable chip RIC; a first control unit 300b can obtain a difference of U2-U1 between the output voltage and the target voltage based on the feedback voltage, at this time, the voltage is adjusted based on the voltage difference U2-U1. If it is adjusted quickly, a second control unit 400b will switch to the closest voltage according to which of the voltages between the target voltage and terminals A, B, C, or D voltageis the closest, then calculating the total voltage to each voltage value, and controlling the first control unit 300b to finely adjust and increase the voltage to a target voltage.

In an implementation of the third embodiment, as shown in Fig. 11, the high-voltage generating circuit for a catheter also comprises a voltage adjustment unit 500b, whose input terminal is connected to the output terminal of each voltage conversion unit, wherein the output terminal of the voltage adjustment unit 500b is connected to the total output positive terminal Vout so as to control the on-off switching between the output terminal of each voltage conversion unit and the total output positive terminal Vout; and wherein a second control unit 400b is also used to control the N voltage conversion units and the voltage adjustment unit 500b based on the target voltage and the adjusted feedback voltage, so as to adjust the output voltage of the total output positive terminal Vout to obtain a target voltage.

Particularly, the first input terminal of the voltage adjustment unit 500b is connected to the first output terminal of the voltage conversion unit 111b and one end of the corresponding energy storage unit 112b; the second input terminal of the voltage adjustment unit 500b is connected to the first output terminal of the voltage conversion unit 121b, the other end of the energy storage unit 112b, and one end of the energy storage unit 122b; the third input terminal of the voltage adjustment unit 500b is connected to the first output terminal of the voltage conversion unit 131b, the other end of the energy storage unit 122b is connected to one end of the energy storage unit 132b; the fourth input terminal of voltage adjustment unit 500b is connected to the first output terminal of voltage conversion unit 141b, the other end of energy storage unit 132b, and one end of energy storage unit 142b.

A second control unit 400b obtains the target voltage of the high-voltage generating circuit and the aforementioned adjusted feedback voltage, and controls the voltage conversion units 111b, 121b, 131b, 141b and the voltage adjustment unit 500b based on the target voltage and the adjusted feedback voltage, so as to quickly adjust the output voltage of the total output terminal to be a target voltage. For example, when the output voltage needs to be quickly adjusted to a target voltage, a first control unit 300b can control a voltage feedback unit 200b to adjust the feedback voltage, so that there is a large difference between the output voltage corresponding to the adjusted feedback voltage and the target voltage; and based on the target voltage, a second control unit 400b can determine the voltage conversion unit that currently needs to work or the energy storage unit that needs to be charged and discharged, and the voltage conversion unit that needs to be connected to the total output positive terminal Vout; assuming that the target voltage is 2000V , then the voltage conversion unit 111b needs to be connected to the total output terminal Vout, and at the same time, the four voltage conversion units are controlled according to the large difference so as to charge the energy storage units, and the charging speed of the energy storage units is adjusted to realize a quick adjustment of the output voltage to be a target voltage. On the contrary, when the output voltage needs to be slowly adjusted to the target voltage of 2000V, a first control unit 300b can control a voltage feedback unit 200b to adjust the feedback voltage, so that there is a small difference between the output voltage corresponding to the adjusted feedback voltage and the target voltage; and based on the small difference, a second control unit 400b can control the four voltage conversion units simultaneously or one by one so as to charge the energy storage units, and adjust the charging speed of the energy storage units to realize a slow adjustment of the output voltage to be a target voltage.

For another example, when the output voltage needs to be quickly adjusted to a target voltage of 300V, a first control unit 300b can control a voltage feedback unit 200b to adjust the feedback voltage, so that there is a large difference between the output voltage corresponding to the adjusted feedback voltage and the target voltage, and a second control unit 400b determines that the voltage conversion unit 141b needs to be connected to the total output terminal Vout based on the target voltage, and at the same time controls the voltage conversion unit to charge the energy storage unit according to the large difference, and adjusts the charging speed of the energy storage units, thereby realizing a rapid adjustment of the output voltage to be a target voltage. On the contrary, when the output voltage needs to be slowly adjusted to a target voltage of 300V, a first control unit 300b can control a voltage feedback unit 200b to adjust the feedback voltage, so that there is a small difference between the output voltage corresponding to the adjusted feedback voltage and the target voltage; and a second control unit 400b can control the voltage conversion units to charge the energy storage units based on the small difference, and adjust the charging speed of the energy storage units, thereby realizing a slow adjustment of the output voltage to be a target voltage.

As a result, the output voltage can be quickly switched to a target voltage through using multiple voltage conversion units, voltage feedback units, and voltage adjustment units, thereby improving the application prospects of pulsed electric field ablation technology in arrhythmia treatment.

Further, N voltage conversion units cooperate with N energy storage units to form N voltage intervals, and a second control unit 400b is specifically used for: obtaining a voltage interval corresponding to the target voltage, and controlling the N voltage conversion units and the voltage adjustment unit 500b based on the voltage interval corresponding to the target voltage and the adjusted feedback voltage.

Particularly, continuing to refer to Fig. 11, four voltage conversion units and four energy storage units can cooperate to form four voltage intervals; assuming that the highest voltages at points A, B, C and D are 2000V, 1500V, 1000V and 500V respectively, then the four voltage intervals formed are [0V, 500V], [500V, 1000V], [1000V, 1500V] and [1500V, 2000V].

When the output voltage needs to be quickly adjusted to a target voltage, a first control unit 300b can control a voltage feedback unit 200b to adjust the feedback voltage, so that there is a large difference between the output voltage corresponding to the adjusted feedback voltage and the target voltage; and a second control unit 400b can determine the corresponding voltage interval based on the target voltage, and then determine the voltage conversion unit that currently needs to work or the energy storage unit that needs to be charged and discharged, as well as the voltage conversion unit that needs to be connected to the total output positive terminal Vout according to the voltage interval. Assuming that the current target voltage is 2000V and the corresponding voltage interval is [1500V, 2000V], then the voltage conversion unit 111b needs to be connected to the total output terminal Vout, and at the same time, based on the large difference, the four voltage conversion units are controlled to charge the energy storage units, and adjust the charging speed of the energy storage units, thereby realizing rapid adjustment of the output voltage to a target voltage. On the contrary, when the output voltage needs to be slowly adjusted to a target voltage of 2000V, a first control unit 300b can control a voltage feedback unit 200b to adjust the feedback voltage, so that there is a small difference between the output voltage corresponding to the adjusted feedback voltage and the target voltage; and a second control unit 400b can control the four voltage conversion units simultaneously or one by one to charge the energy storage units based on the small difference, and adjust the charging speed of the energy storage units, thereby realizing a slow adjustment of the output voltage to a target voltage. As a result, the output voltage can be quickly adjusted to the target voltage.

In an implementation of the third embodiment, as shown in Fig. 11, the high-voltage generating circuit for a catheter further comprises: a current adjustment unit 600b, whose input terminal is connected to an output terminal of the current voltage adjustment unit 500b, wherein an output terminal of the current adjustment unit 600b is connected to the total output positive terminal Vout, so as to adjust the output current of the total output positive terminal Vout; and a second control unit 400b is also used to obtain a target power of the high-voltage generating circuit, and controls the N voltage conversion units, the voltage adjustment unit 500b and the current adjustment unit 600b based on the target power and the adjusted feedback voltage, so as to adjust the output voltage and output current of the total output positive terminal Vout to obtain a target power.

Particularly, the second control unit 400b can control the current adjustment unit 600b to change the output current of the total output terminal by changing the size of the built-in resistor of the current adjustment unit 600b. For example, the current adjustment unit 600b can be a variable resistor with infinitely adjustable resistance, by changing the resistance value of the variable resistor, the output current can be directly adjusted to the required current, thereby achieving a rapid switching of different output currents. Further, the second control unit 400b can realize flexible adjustment of the output voltage, output current and output power by controlling the N voltage conversion units, the voltage adjustment unit 500b and the current adjustment unit 600b. Particularly, when only a target power is required, the output voltage and/or output current of the total output terminal can be adjusted through the aforementioned methods, so that the output power reaches the target power; when there are requirements for both a target power and a target voltage, the output voltage of the total output terminal can be adjusted through the aforementioned method to reach the target voltage, and then the output current can be changed by adjusting the internal resistance of the current adjustment unit 600b, so that the output power reaches the target power.

It should be noted that, a second control unit 400b can also control the N voltage conversion units, the voltage adjustment unit 500b and the current adjustment unit 600b, thereby realizing a rapid switching of the target power. For example, when only a target power is required, assuming that the target power is 1000w, and the target voltage before switching is 800V, if the output current can reach 1.25A, then a quick adjustment of the target power can be achieved by adjusting the output current. Compared to a solution with only one voltage conversion unit and corresponding energy storage unit, the process of adjusting the output voltage is eliminated, and the adjustment of the output voltage involves the charge and discharge process, as a result, the adjustment speed of the target power with the current adjustment unit 600b makes it possible to be faster and more energy efficient. When there are requirements for both a target power and a target voltage, this embodiment can achieve the output of any target voltage and any target power within the range, and there may be no constraint relationship between the two, for example, as for a solution with only one voltage conversion unit and the corresponding energy storage unit, the target power and target voltage are in a corresponding relationship, and cannot be adjusted flexibly.

Therefore, rapid and flexible switching of different output voltages, output currents, and output powers can be achieved through multiple voltage conversion units, voltage feedback units, voltage adjustment units, and current adjustment units.

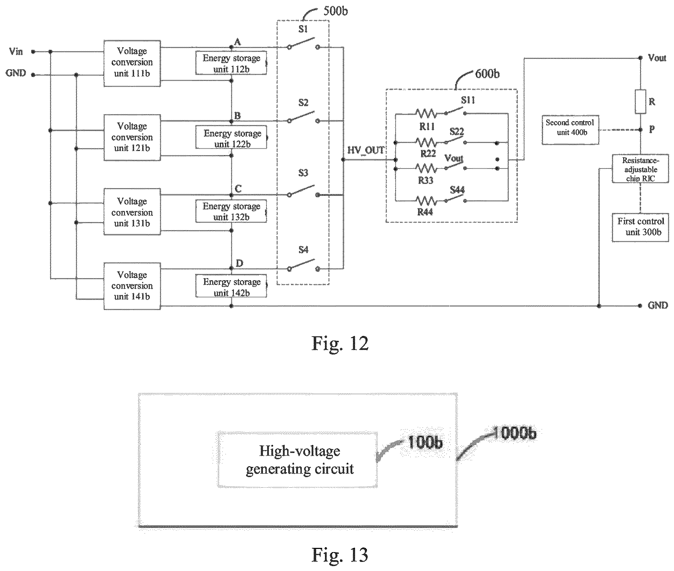

In an implementation of the third embodiment, as shown in Fig. 12, the voltage adjustment unit 500b comprises: N first switches, which correspond one-to-one to the N voltage conversion units, and each first switch is connected in series between an output terminal of the corresponding voltage conversion unit and the total output positive terminal Vout.

Particularly, the voltage adjustment unit 500b comprises four first switches, namely first switches S1, S2, S3 and S4. The first switches S1, S2, S3 and S4 are respectively connected in series between an output terminal of the corresponding voltage conversion unit and the total output positive terminal Vout. The voltage output of a corresponding voltage conversion unit is realized by controlling the on-off switching of the first switches S1, S2, S3 and S4, thereby realizing a rapid switching of the output voltage. For example, when the first switch S4 is turned on, and the other first switches are turned off, then the output voltage is VO; when the first switch S1 is turned on, and the other first switches are turned off, then the output voltage is 4VO, thereby realizing a rapid switching of the output voltage from VO to 4VO, and expanding the output voltage range.

In an implementation of the third embodiment, as shown in Fig. 12, the current adjustment unit 600b comprises M current adjustment branches connected in parallel, wherein M is an integer greater than 1, and each of the current adjustment branches comprises: a current-limiting resistor (such as R11), whose one end is connected to an output terminal of the voltage adjustment unit 500b; and a current-limiting switch (such as S11), whose one end is connected to the other end the current-limiting resistor (such as R11), and the other end of the current limiting switch (such as S11) is connected to the total output positive terminal Vout.

Particularly, four current adjustment branches are taken as examples. The first current adjustment branch comprises: a current limiting resistor R11 and a current limiting switch S11; wherein one end of the current limiting resistor R11 is connected to the output terminal of the voltage adjustment unit 500b, one end of the current-limiting switch S11 is connected to the other end of the current-limiting resistor R11, and the other end of the current-limiting switch S11 is connected to the total output positive terminal Vout. The second current adjustment branch comprises: a current-limiting resistor R22 and a current-limiting switch S22; wherein one end of the current-limiting resistor R22 is connected to the output terminal of the voltage adjustment unit 500b, one end of the current-limiting switch S22 is connected to the other end of the current-limiting resistor R22, and the other end of the current-limiting switch S22 is connected to the total output positive terminal Vout. The third current adjustment branch comprises: a current-limiting resistor R33 and a current-limiting switch S33; wherein one end of the current-limiting resistor R33 is connected to the output terminal of the voltage adjustment unit 500b, one end of the current-limiting switch S33 is connected to the other end of the current-limiting resistor R33, and the other end of the current-limiting switch S33 is connected to the total output positive terminal Vout. The fourth current adjustment branch comprises: a current-limiting resistor R44 and a current-limiting switch S44; wherein one end of the current-limiting resistor R44 is connected to the output terminal of the voltage adjustment unit 500b, one end of the current-limiting switch S44 is connected to the other end of the current-limiting resistor R44, and the other end of the current limiting switch S44 is connected to the total output positive terminal Vout. It should be noted that, the resistance values of the current limiting resistors R11, R22, R33 and R44 can be the same or different.

When the high-voltage generating circuit is operating, as shown in Fig. 12, the first to fourth current adjustment branches are connected in parallel, and the voltage obtained by each current adjustment branch is HV_OUT. The on-off status of different current adjustment branches are controlled by controlling the on-off switching of the current limiting switches in the current adjustment branches, so as to adjust the output current of the total output positive terminal Vout based on the current-limiting resistance of the conducting current adjustment branch. For example, when the voltage HV_OUT input to the current adjustment unit 600b remains unchanged, if the current-limiting switch S11 is turned on, and the other current-limiting switches are turned off, then the output current is the ratio of the input voltage HV_OUT to the current-limiting resistor R11; alternatively, if the current-limiting switches S11 and S22 are turned on, and the other current-limiting switches are turned off, then the output current is the ratio of the input voltage HV_OUT to the current-limiting resistors R11 and R22 connected in parallel. Therefore, the output current can be switched quickly, that is to say, the output current of the total output positive terminal Vout can be adjusted by selecting the current-limiting resistors with different resistance values, or selecting different numbers of current-limiting resistors.

In an implementation of the third embodiment, each of the voltage conversion units is a flyback conversion circuit, a forward conversion circuit, an LLC resonance circuit, a push-pull circuit or a bridge circuit. That is to say, a voltage conversion unit in this application can be a flyback conversion circuit, a forward conversion circuit, an LLC resonance circuit, a push-pull circuit or a bridge circuit, etc. The type of the voltage conversion unit must be consistent during use, and the specific method to be used can be selected and set according to actual needs.

To sum up, in the high-voltage generating circuit for a catheter according to an embodiment of the present application, the input terminals of the voltage conversion units are connected in parallel and then connected to the total input terminal of the high-voltage generating circuit, and the output terminals of the voltage conversion units are connected in series and then connected to the total output terminal of the high-voltage generating circuit, so as to detect the output voltage of the total output terminal to obtain the feedback voltage and adjust the feedback voltage. A first control unit is connected to the adjustment terminal of the voltage feedback unit, so as to control the voltage feedback unit to adjust the voltage; and a second control unit is used to obtain a target voltage of the high-voltage generating circuit, and control the N voltage conversion units based on the target voltage and the adjusted feedback voltage, so as to adjust the output voltage of the total output terminal to obtain a target voltage. Therefore, the output voltage can be quickly switched to a target voltage through multiple voltage conversion units and voltage feedback units, thereby improving the application prospects of pulsed electric field ablation technology in arrhythmia treatment.

Fig. 13 is a schematic structural diagram of an ablation tool according to the fourth embodiment of the present application. Referring to Fig. 13, the ablation tool 1000b comprises the above-mentioned high-voltage generating circuit 100b for a catheter.

According to the ablation tool according to an embodiment of the present application, through the above-mentioned high-voltage generating circuit for a catheter, the output voltage can be quickly switched to a target voltage through multiple voltage conversion units and voltage feedback units, thereby improving the application prospects of pulsed electric field ablation technology in arrhythmia treatment.

When the high-voltage generating circuit 100b for a catheter is such a high-voltage generating circuit in which a voltage feedback unit 200b, a first control unit 300b, and a second control unit 400b, etc. are arranged in the high-voltage generating circuit 100a for the catheter according to the first embodiment of present application, then accordingly, the ablation tool 1000b can also have the advantages of broadening the output range of the voltage, preventing the output voltage from being too high, and preventing overvoltage damage to electronic components.

It should be noted that, the logic and/or steps represented in the flowcharts or otherwise described herein, for example, may be considered to be a fixed order of executable instructions for implementing logical functions, which may be embodied in any computer-readable medium for use by, or in conjunction with, an instruction execution system, apparatus, or device (such as a computer-based system, a system comprising a processor, or other system that can retrieve and execute instructions from such instruction execution system, apparatus, or device). For the purposes of this specification, a "computer-readable medium" may be any device that can contain, store, communicate, transmit, or transfer a program for use by or in connection with an instruction execution system, apparatus, or device. More particular examples (non-exhaustive list) of computer readable media comprise the following: an electrical connection with one or more wires (electronic device), a portable computer disk cartridge (magnetic device), a random access memory (RAM), a read-only memory (ROM), an erasable and programmable read-only memory (EPROM or flash memory), a fiber optic device, and a portable compact disc read-only memory (CDROM). Additionally, the computer-readable media may even be paper or other suitable media on which the program may be printed, as the paper or other medium for example, may be optically scanned, and subsequently edited, interpreted, or processed with other suitable manner as necessary to obtain the program electronically, and then store it in computer memory.

It should be understood that, various parts of the present application may be implemented by hardware, software, firmware, or a combination thereof. In the above embodiments, various steps or methods may be implemented by software or firmware stored in a memory and executed by a suitable instruction execution system. For example, if it is implemented by hardware, as in another embodiment, it may be implemented by any one or a combination of the following technologies commonly known in the art: discrete logic circuit of logic gate circuit for implementing a logic function on a data signal, application-specific integrated circuit with suitable combinational logic gate circuit, programmable gate array (PGA), field programmable gate array (FPGA), etc.

In the description of this specification, reference terms "one embodiment," "some embodiments," "an example," "a particular example," or "some examples" or the likes mean that a specific feature, structure, material or characteristic described in conjunction with such an embodiment or example is encompassed in at least one embodiment or example of the application. In this specification, schematic representations of the above terms do not necessarily refer to the same embodiment or example. Furthermore, the specific feature, structure, material or characteristic described may be combined in any suitable manner in any one or more embodiments or examples.

In addition, the terms "a first" and "a second" are used for descriptive purposes only, and cannot be understood as indicating or implying relative importance, or implicitly indicating quantity of the indicated technical features. Therefore, a feature defined as "a first" or "a second" may explicitly or implicitly comprise at least one of these features. In the description of the present application, "multiple" means at least two, such as two, three, etc., unless otherwise expressly and specifically defined.

In the present application, unless otherwise clearly stated and defined, the terms "installation", "connection", "connected", "fixed" and other terms should be understood in a broad sense. For example, it may be a fixed connection or a detachable connection, or integrated into one; it may be a mechanical connection or an electrical connection; it may be a direct connection or an indirect connection through an intermediate medium; it may be an internal connection between two elements or an interactive relationship between two elements, unless otherwise clearly specified. For those of ordinary skilled in the art, the specific meanings of the above terms in the present application may be understood according to specific circumstances.

Although the embodiments of the present application have been shown and described above, it may be understood that the above embodiments are illustrative, and should not be construed as limitations of the present application. Those of ordinary skilled in the art can make transformation, modifications, substitutions or variations to the above embodiments within the scope of the present application.

## Claims

1. A high-voltage generating circuit for a catheter, comprising:
N voltage conversion units and N energy storage units, wherein the input terminals of the N voltage conversion units are connected in parallel and then connected to a total input terminal of the high-voltage generating circuit, the output terminals of the N voltage conversion units are connected in series and then connected to a total output terminal of the high-voltage generating circuit; the N energy storage units correspond one-to-one to the N voltage conversion units, and each energy storage unit is connected between the output terminals of the corresponding voltage conversion units, and each of the N voltage conversion units is used to perform voltage conversion on the input voltage of the total input terminal and charge the corresponding energy storage unit, wherein N is an integer greater than 1; and
wherein the total output terminal of the high-voltage generating circuit can be connected to one or more of the energy storage units connected in series so as to output different voltages.

2. The high-voltage generating circuit for a catheter according to claim 1, further comprising:
a voltage adjustment unit, whose input terminal is connected to an output terminal of each voltage conversion unit, wherein an output terminal of the voltage adjustment unit is connected to the total output terminal so as to control the on-off switching between the output terminal of each voltage conversion unit and the total output terminal; and
a control unit, which is connected to the N voltage conversion units and the voltage adjustment unit so as to obtain a target voltage of the high-voltage generating circuit, wherein the N voltage conversion units and the voltage adjustment unit are controlled according to the target voltage so as to adjust the output voltage of the total output terminal to be a target voltage.

3. The high-voltage generating circuit for a catheter according to claim 2, wherein the N voltage conversion units cooperate with the N energy storage units to form N voltage intervals, and the control unit is specifically used for: obtaining a voltage interval corresponding to the target voltage, and wherein the N voltage conversion units and the voltage adjustment unit are controlled according to voltage interval corresponding to the target voltage.

4. The high-voltage generating circuit for a catheter according to claim 2, further comprising:
a current adjustment unit, whose input terminal is connected to an output terminal of the voltage adjustment unit, wherein an output terminal of the current adjustment unit is connected to the total output terminal so as to adjust the output current of the total output terminal; and
wherein the control unit is also connected to the current adjustment unit so as to obtain a target power of the high-voltage generating circuit; the N voltage conversion units, the voltage adjustment unit and the current adjustment unit are controlled according to the target power of the high-voltage generating circuit, so that the output voltage and output current of the total output terminal are adjusted to obtain the target power.

5. The high-voltage generating circuit for a catheter according to any one of claims 2-4, wherein the N energy storage units have the same structure, and each energy storage unit comprises an energy storage capacitor which is connected in parallel between the output terminals of the corresponding voltage conversion units.

6. The high-voltage generating circuit for a catheter according to any one of claims 2-4, wherein the voltage adjustment unit comprises: N first switches which correspond one-to-one to the N voltage conversion units, and each first switch is connected in series between an output terminal of the corresponding voltage conversion unit and the total output terminal.

7. The high-voltage generating circuit for a catheter according to claim 6, wherein the N first switches have the same structure, and each first switch comprises:
a relay, wherein one end of the switch of the relay is connected to an output terminal of the corresponding voltage conversion unit, and one end of the coil of the relay is connected to the preset power supply;
a voltage stabilizing tube, wherein its anode is connected to the other end of the coil of the relay and its cathode is connected to said one end of the coil of the relay;
a second diode, wherein its anode is connected to the other end of the switch of the relay and its cathode is connected to the total output terminal;
a second switch tube, wherein its first end is connected to the other end of the coil of the relay, and a second end of the second switch tube is connected to ground, and wherein the control terminal of the second switch tube is connected to the control unit.

8. The high-voltage generating circuit for a catheter according to claim 4, wherein the current adjustment unit comprises M current adjustment branches connected in parallel, M is an integer greater than 1, and each current adjustment branch comprises:
a current-limiting resistor, one end of which is connected to an output terminal of the voltage adjustment unit; and
a current-limiting switch, one end of which is connected to the other end of the current-limiting resistor, wherein the other end of the current-limiting switch is connected to the total output terminal.

9. The high-voltage generating circuit for a catheter according to any one of claims 2-4, wherein the N voltage conversion units have the same structure, each voltage conversion unit comprises a switch tube for voltage conversion, and some or all of the N voltage conversion units share the switch tube.

10. The high-voltage generating circuit for a catheter according to claim 9, wherein each of the voltage conversion units is a flyback conversion circuit, a forward conversion circuit, an LLC resonance circuit, a push-pull circuit or a bridge circuit.

11. The high-voltage generating circuit for a catheter according to any one of claims 2-4, wherein the control unit comprises one or more control chips, when the control unit comprises one control chip, the N voltage conversion units share the control chip; or when the control unit comprises multiple control chips, some of the N voltage conversion units share one control chip, or each voltage conversion unit corresponds to one of the control chips.

12. The high-voltage generating circuit for a catheter according to claim 11, further comprising:
an overvoltage protection unit, which comprises voltage stabilizing tubes connected in parallel with each of the energy storage units.

13. The high-voltage generating circuit for a catheter according to claim 12, wherein the overvoltage protection unit also comprises an optical coupler, each of the optical couplers corresponds to one control chip;
wherein in each voltage conversion unit controlled by the control chip and the corresponding energy storage unit, at least two or more voltage stabilizing tubes are connected in parallel to at least an energy storage unit closest to the low voltage end, a light emitter of the optical coupler is connected in parallel with part of the voltage stabilizing tubes, and an output terminal of the light receptor of the optical coupler is connected to the under-voltage protection pin of the control chip.

14. The high-voltage generating circuit for a catheter according to claim 1, further comprising:
a voltage feedback unit, whose detection end is connected to the total output terminal so as to detect the output voltage of the total output terminal to obtain a feedback voltage and adjust the feedback voltage;
a first control unit, which is connected to the adjustment terminal of the voltage feedback unit so as to control the voltage feedback unit to adjust the feedback voltage;
a second control unit, which is connected to the output terminals of the N voltage conversion units and the voltage feedback unit so as to obtain a target voltage of the high-voltage generating circuit, wherein the N voltage conversion units are controlled based on the target voltage and the adjusted feedback voltage, so as to adjust the output voltage of the total output terminal to obtain a target voltage.

15. The high-voltage generating circuit for a catheter according to claim 14, wherein the voltage feedback unit comprises:
a voltage sampling subunit, wherein its one end is connected to the total output terminal;
a voltage adjustment subunit, wherein its one end is connected to the other end of the voltage sampling subunit and forms a first node, the first node is connected to the second control unit, and the other end of the voltage adjustment subunit is connected to the first control unit.

16. The high-voltage generating circuit for a catheter according to claim 15, wherein:
the voltage sampling subunit comprises: a sampling resistor, whose one end is connected to the total output terminal;
the voltage adjustment subunit comprises: a resistance-adjustable chip, whose first end is connected to the other end of the sampling resistor, wherein a second end of the resistance-adjustable chip is connected to ground, and the control terminal of the resistance-adjustable chip is connected to the first control unit.

17. The high-voltage generating circuit for a catheter according to claim 16, wherein the first control unit is specifically used to send an adjustment instruction of feedback voltage to the resistance-adjustable chip, so that the resistance-adjustable chip can obtain an adjustment amount of resistance according to the adjustment instruction of feedback voltage, and then adjust its own resistance based on the adjustment amount of resistance.

18. The high-voltage generating circuit for a catheter according to any one of claims 14-17, further comprising:
a voltage adjustment unit, whose input terminal is connected to the output terminal of each voltage conversion unit, wherein the output terminal of the voltage adjustment unit is connected to the total output terminal so as to control the on-off switching between the output terminal of each voltage conversion unit and the total output terminal; and
wherein the second control unit is also used to control the N voltage conversion units and the voltage adjustment unit based on the target voltage and the adjusted feedback voltage, so as to adjust the output voltage of the total output terminal to obtain a target voltage.

19. The high-voltage generating circuit for a catheter according to claim 18, wherein the N voltage conversion units cooperate with the N energy storage units to form N voltage intervals, and the second control unit is specifically used for: obtaining a voltage interval corresponding to the target voltage, and wherein the N voltage conversion units and the voltage adjustment unit are controlled according to voltage interval corresponding to the target voltage and the adjusted feedback voltage.

20. The high-voltage generating circuit for a catheter according to claim 18, further comprising:
a current adjustment unit, whose input terminal is connected to the output terminal of the voltage adjustment unit, wherein the output terminal of the current adjustment unit is connected to the total output terminal so as to adjust the output current of the total output terminal; and
wherein the second control unit is also used to obtain a target power of the high-voltage generating circuit; wherein the N voltage conversion units, the voltage adjustment unit and the current adjustment unit are controlled according to the target power and the adjusted feedback voltage, so that the output voltage and output current of the total output terminal are adjusted to obtain the target power.

21. The high-voltage generating circuit for a catheter according to claim 18, wherein the voltage adjustment unit comprises: N first switches, the N first switches correspond one-to-one to the N voltage conversion units, and each first switch is connected in series between the output terminal of the corresponding voltage conversion unit and the total output terminal.

22. The high-voltage generating circuit for a catheter according to claim 20, wherein the current adjustment unit comprises M current adjustment branches connected in parallel, M is an integer greater than 1, and each current adjustment branch comprises:
a current-limiting resistor, one end of which is connected to an output terminal of the voltage adjustment unit; and
a current-limiting switch, one end of the current-limiting switch is connected to the other end of the current-limiting resistor, and the other end of the current-limiting switch is connected to the total output terminal.

23. The high-voltage generating circuit for a catheter according to claim 14, wherein each of the voltage conversion units is a flyback conversion circuit, a forward conversion circuit, an LLC resonance circuit, a push-pull circuit or a bridge circuit.

24. An ablation tool, wherein it comprises a catheter and the high-voltage generating circuit for a catheter according to any one of claims 1-23.
